(19) Europäisches Patentamt
European Patent Office
Office européen des brevets

(11) **EP 3 070 476 A1**

(12) **EUROPÄISCHE PATENTANMELDUNG**

(43) Veröffentlichungstag:
**21.09.2016 Patentblatt 2016/38**

(51) Int Cl.:
***G01N 33/74*** *(2006.01)*    ***C07K 14/575*** *(2006.01)*

(21) Anmeldenummer: **15159303.5**

(22) Anmeldetag: **16.03.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **Mediagnost Gesellschaft für Forschung und Herstellung von Diagnostika GmbH 72770 Reutlingen (DE)**

(72) Erfinder: **Flehmig, Bertram 72076 Tübingen (DE)**

(74) Vertreter: **Louis Pöhlau Lohrentz Patentanwälte Postfach 30 55 90014 Nürnberg (DE)**

(54) **ZUSAMMENSTELLUNG VON NACHWEISREAGENZIEN, IN-VITRO-VERFAHREN ZUM NACHWEIS VON MUTIERTEM LEPTIN UND VERWENDUNG EINES NACHWEISREAGENZES**

(57)    Die Erfindung betrifft eine Zusammenstellung von Nachweisreagenzien, wobei die Zusammenstellung ein erstes und ein zweites Nachweisreagenz umfasst, wobei das erste Nachweisreagenz nichtmutiertes Leptin mit einem ersten Bindungswert, jedoch mutiertes Leptin nicht oder maximal mit 50 % des Bindungswerts von nichtmutiertem Leptin bindet, und wobei das zweite Nachweisreagenz sowohl mutiertes als auch nichtmutiertes Leptin mit einem zweiten Bindungswert bindet. Die Erfindung betrifft ferner ein In-vitro-Verfahren zum Nachweis von mutiertem Leptin und die Verwendung eines Nachweisreagenzes.

## Beschreibung

Die vorliegende Erfindung betrifft eine Zusammenstellung von Nachweisreagenzien, ein In-vitro-Verfahren zum Nachweisen von mutiertem Leptin sowie die Verwendung eines Nachweisreagenzes, das nicht mutiertes Leptin, jedoch mutiertes Leptin nicht oder maximal mit 50 % des Bindungswerts von nichtmutiertem Leptin bindet, bei der Diagnose, insbesondere bei der Differenzialdiagnose, von Fettleibigkeit.

Fettleibigkeit, die auch als Adipositas oder Obesitas bezeichnet wird, ist eine Ernährungs- und Stoffwechselkrankheit, bei der die betroffenen Personen ein deutliches Übergewicht aufweisen. Das Übergewicht ist insbesondere auf das Bilden von Körperfett zurückzuführen. Hauptbestandteil des Fettgewebes sind dabei Adipozyten, wobei zwischen univakuolären Adipozyten und plurivakuolären Adipozyten unterschieden wird.

Die univakuolären Adipozyten verfügen nur über eine mit Lipiden gefüllte Vakuole. Dieser Zelltyp baut das so genannte weiße Fettgewebe auf. Bei den plurivakuolären Adipozyten werden die Lipide in mehreren voneinander getrennten Vakuolen gespeichert. Die plurivakuolären Adipozyten bauen hauptsächlich das so genannte braune Fettgewebe auf.

Die Adipozyten enthalten das Obese-Gen, welches für Leptin kodiert. In geringeren Mengen wird das Leptin auch in der Plazenta, der Magenschleimhaut, dem Knochenmark, dem Brustepithel, der Skelettmuskulatur, der Hypophyse sowie dem Hypothalamus erzeugt.

Das Leptin hemmt das Auftreten von Hungergefühlen und spielt bei der Regulierung des Fettstoffwechsels von Säugetieren, insbesondere von Menschen, eine bedeutende Rolle. Das Leptin wird dabei hauptsächlich von den Adipozyten des weißen Fettgewebes sekretiert. Der Leptinpegel ist dabei positiv mit der Körperfettmenge korreliert.

Bei dem humanen Leptin handelt es sich um ein Protein, das einschließlich Signalpeptid aus 167 Aminosäuren aufgebaut ist. Das N-terminale Signalpeptid weist eine Länge von 21 Aminosäuren auf und wird bei dem Sekretionsvorgang abgespalten, so dass das reife Leptin 146 Aminosäuren aufweist. Das Leptin wirkt durch Bindung an spezifische Leptinrezeptoren, die sowohl im Gehirn als auch in peripheren Geweben vorhanden sind. Aufgrund von alternativen Spleißvorgängen gibt es mehrere Isoformen des Leptinrezeptors. Die Isoform A ist eine kurze Leptinrezeptor-Isoform und spielt eine bedeutende Rolle beim Transport von Leptin über die Blut-Hirn-Schranke. Die Isoform B, bei der es sich um die sogenannte lange Leptin-Isoform handelt, bewirkt die Signaltransduktion und wird im Hypothalamus exprimiert.

Bei Bindung von Leptin an die Isoform B des Leptinrezeptors werden mehrere Signaltransduktionswege, einschließlich der Januskinase (JAK) und der STAT-Proteine (STAT: Signal Transducers and Activators of Transcription), insbesondere STAT-3, und der Phosphatidylinositol-3-kinase (PI3K), aktiviert. Bei anderen Signalwegen wird die Mitogen-aktivierte Proteinkinase (MAPK), 5'-Adenosin-Monophosphat-aktivierte Proteinkinase (AMPK) und das Ziel des Rapamycins im Säugetier (mTOR: mechanistic Target of Rapamycin oder auch mammalian target of rapamycin) aktiviert.

Homozygote Mutationen im Leptin-Gen führen zu einem vollständigen Leptin-Mangel, wie er in äußerst seltenen Fällen von humaner Fettleibigkeit beschrieben ist.

Die weitaus größere Mehrheit von fettleibigen Menschen weist jedoch hohe zirkulierende Leptinpegel auf (Kelesidis T. et al., Ann Intern Med. 2010, January 19, 152(2): 93-100, Narrative Review: The Role of Leptin in Human Physiology: Emerging Clinical Applications).

Patienten, die keinen nachweisbaren Leptinpegel bei Lipodystrophie, hypothalamischer Amenorrhoe und kongenitaler Leptindefizienz (CLD) aufweisen, werden mit einer Leptinersatztherapie und Gabe von Metreleptin behandelt. Bei Metreleptin handelt es sich um ein rekombinantes Leptin-Analogon, das aus 147 Aminosäuren des reifen humanen Leptins zuzüglich eines Methioninrestes am N-Terminus aufgebaut ist. Es handelt sich dabei um ein nichtglykolysiertes Polypeptid mit einer Disulfid-Bindung zwischen einer Cys-97 und Cys-147. Das Molekulargewicht beträgt etwa 16,15 kDa.

Metreleptin ist von der FDA (Food and Drug Administration, USA) zur Behandlung von Patienten, die teilweise an Lipodystrophie erkrankt sind, jedoch nicht zugelassen, weil es keine Nachweise gibt, dass bei einer heterogenen Gruppe Metreleptin sicher und wirksam ist. Metreleptin weist mithin eine sehr begrenzte Bedeutung bei der Behandlung von Patienten mit herkömmlicher Fettleibigkeit auf, da diese Patienten einen hohen Pegel an Leptin aufweisen und mithin eine zentrale Leptinresistenz zeigen (Paz-Filho G. et al., Metabolism (2014), 1-11, Leptin treatment: Facts and expectations).

Mutationen in dem für Leptin kodierenden Gen führen üblicherweise zur Abwesenheit von zirkulierendem Leptin und mithin zu extremer Fettleibigkeit. Wabitsch et al. (N Engl J Med; 372; 1; January 1, 2015; 48-54) haben bei einem zweijährigen Jungen mit extremer Fettleibigkeit eine homozygote Mutation gefunden, die zum Austausch von Guanin gegen Thymin in der Position 298 führt. In dem translatierten Leptin führt dies zu einem Aminosäureaustausch von Asparaginsäure gegen Tyrosin in der Sequenzposition 100 des Leptins (unter Berücksichtigung des Signalpeptids mit den Aminosäuren 1 bis 21). Das mutierte Protein wird sekretiert, bindet jedoch nicht an den Leptinrezeptor und aktiviert diesen auch nicht. Bei Behandlung des Patienten mit Metreleptin kam es zu einer Normalisierung des Essverhaltens und zu einem Gewichtsverlust.

Der Leptinpegel im Blut kann beispielsweise mittels RIA (Radioimmuoassay) oder ELISA (Enzyme-linked Immunoassay) bestimmt werden (Kratzsch et al., Horm Res 2002; 57: 127-132: A Rapid Quantitative Immunofunctional Assay for Measuring Human Leptin).

Aufgabe der vorliegenden Erfindung ist es, ein Verfahren bereitzustellen, das eine verbesserte Differenzialdiagnose bei Fettleibigkeit erlaubt. Des Weiteren ist es Aufgabe der Erfindung, Mittel zur Durchführung eines solchen Verfahrens bereitzustellen.

Die der Erfindung zugrunde liegende Aufgabe wird durch Bereitstellung einer Zusammenstellung von Nachweisreagenzien gelöst, wobei die Zusammenstellung ein erstes und ein zweites Nachweisreagenz umfasst, wobei das erste Nachweisreagenz nichtmutiertes Leptin mit einem ersten Bindungswert, jedoch mutiertes Leptin nicht oder maximal mit 50 % des Bindungswerts von nichtmutiertem Leptin bindet, und wobei das zweite Nachweisreagenz sowohl mutiertes als auch nichtmutiertes Leptin mit einem zweiten Bindungswert bindet.

Die der Erfindung zugrunde liegende Aufgabe wird des Weiteren durch ein Verfahren zum Nachweis von mutiertem Leptin in vitro gelöst, wobei das Verfahren folgende Schritte umfasst:

- Bestimmen der Bindung von nichtmutiertem Leptin aus einem, vorzugsweise ersten, flüssigen Probenmaterial an ein erstes Nachweisreagenz unter Erhalt eines ersten Bindungswerts,
- Bestimmen der Bindung sowohl von mutiertem Leptin als auch von nichtmutiertem Leptin aus einem, vorzugsweise zweiten, flüssigen Probenmaterial an ein zweites Nachweisreagenz unter Erhalt eines zweiten Bindungswerts,

wobei mutiertes Leptin vorhanden ist, wenn der erste Bindungswert kleiner als der zweite Bindungswert ist.

Das vorzugsweise erste und das vorzugsweise zweite flüssige Probenmaterial sind vor der Durchführung des erfindungsgemäßen Verfahrens von demselben Subjekt isoliert worden.

Die der Erfindung zugrunde liegende Aufgabe wird auch Verwendung eines Nachweisreagenzes, das nichtmutiertes Leptin mit einem ersten Bindungswert, jedoch mutiertes Leptin nicht oder maximal mit 50 % des Bindungswerts von nichtmutiertem Leptin bindet, bei der Diagnose, vorzugsweise Differenzialdiagnose, bei Fettleibigkeit gelöst.

Im Sinne der Erfindung wird unter einer "Zusammenstellung von Nachweisreagenzien" jede Anordnung verstanden, die sowohl das erste als auch das zweite Nachweisreagenz umfasst.

Bei dieser Anordnung kann es sich beispielsweise um ein, vorzugsweise immunologisches, Testsystem handeln. Das immunologische Testsystem kann aus einem RIA (Radioimmunoassay), ELISA (Enzyme-linked Immunoassay), FIA (Fluoreszenz-Immunoassay) oder LIA (Luminescence Immunoassay) ausgewählt werden. Bei dem Testsystem kann es sich jedoch auch um einen Western-Blot, eine Chromatographie, beispielsweise Affinitätschromatographie, einen Zellassay, einen Partikel-basierten Assay, sowie jeweils einen derartigen Multiplex-Assay, oder einen Biochip handeln, die jeweils sowohl das erste als auch das zweite Nachweisreagenz umfassen.

Unter dem "ersten Nachweisreagenz" sowie "zweiten Nachweisreagenz" werden affinitätsbasierte Nachweisreagenzien verstanden. Unter "affinitätsbasierten Nachweisreagenzien" werden erfindungsgemäß beispielsweise Aptamere, z.B. DNA-Aptamere, RNA-Aptamere oder Peptid-Aptamere, monoklonale Antikörper, polyklonale Antikörper, Antikörperfragmente, beispielsweise Fab-, $F(ab)_2$-, Fv- oder scFv-Antikörperfragmente verstanden.

Gemäß einer bevorzugten Weiterbildung werden unter "affinitätsbasierten Nachweisreagenzien" monoklonale Antikörper, polyklonale Antikörper, Antikörperfragmente, beispielsweise Fab-, $F(ab)_2$-, Fv- oder scFv-Antikörperfragmente, verstanden.

Die monoklonalen Antikörper oder Antikörperfragmente können über herkömmliche Verfahren, beispielsweise die Hybridom-Technik oder die Phagen-Display-Technik, hergestellt und durch übliche Screeningverfahren bezüglich ihrer Spezifität selektiert werden.

Des Weiteren kann sowohl das erste als auch das zweite Nachweisreagenz ein Leptinrezeptor sein. Unter einem "Leptinrezeptor" wird erfindungsgemäß jede spezifisch Leptin-bindende Struktur verstanden, beispielsweise natürlich vorkommender Leptinrezeptor oder Leptinrezeptorfragmente. Auch kann es sich bei der Leptinrezeptorstruktur um synthetische oder mutierte natürlich vorkommende Leptinrezeptoren oder Leptinrezeptorfragmente handeln.

Erfindungsgemäß wesentlich ist, dass das erste Nachweisreagenz nichtmutiertes Leptin, jedoch mutiertes Leptin nicht oder nur mit maximal 50% des Bindungswertes von nichtmutiertem Leptin bindet. Vorzugsweise bindet das erste Nachweisreagenz mutiertes Leptin mit maximal 40 %, maximal 30%, maximal 20 %, maximal 10 %, maximal 5 % des Bindungswertes von nichtmutiertem Leptin. Äußerst bevorzugt bindet das erste Nachweisreagenz nichtmutiertes Leptin, jedoch mutiertes Leptin nicht oder nur mit maximal 5%, maximal 2 % des Bindungswertes von nichtmutiertem Leptin. Vorzugsweise bindet das erste Nachweisreagenz kein mutiertes Leptin.

Erfindungsgemäß wird unter "Bindungswert" der für das jeweils verwendete Messsystem charakteristische Messwert für die Bindung von Nachweisreagenz an nichtmutiertes Leptin einerseits bzw. nichtmutiertes und mutiertes Leptin andererseits verstanden. Dieser Messwert kann entweder direkt oder indirekt ein Maß für die Bindungsaffinität sein.

Der die Bindungsaffinität reflektierende Messwert stellt dabei ein Maß für die Bindung des ersten Nachweisreagenzes an nichtmutiertes Leptin dar. Beim zweiten Nachweisreagenzes stellt der die Bindungsaffinität reflektierende Messwert ein Maß für die Bindung von sowohl mutiertem als auch nichtmutiertem Leptin dar.

Der für den Bindungswert stehende Messwert kann beispielsweise die Einheit einer Konzentration (oder einer Menge) haben. So werden beispielsweise beim RIA, ELISA, FIA oder LIA üblicherweise die Konzentration (oder Menge) an bindungsfähigem nichtmutiertem Leptin einerseits oder die Konzentration (oder Menge) an bindungsfähigem nichtmutiertem und mutiertem Leptin andererseits in einer Messprobe bestimmt.

Der für den Bindungswert stehende Messwert kann auch als Gleichgewichtskonstante $K_D$, d.h. als chemisches Gleichgewicht im Sinne des Massenwirkungsgesetzes, ausgedrückt werden.

Gemäß einer Variante der Erfindung wird die Bindungsaffinität mit der Gleichgewichtskonstante $K_D$ korreliert:

$$K_D = (C_N \cdot C_L)/C_{NL,}$$

wobei:

$C_N$ die Konzentration des Nachweisreagenzes,
$C_L$ die Konzentration von (nichtmutiertem oder nichtmutiertem und mutiertem) Leptin, und
$C_{NL}$ die Konzentration des Komplexes von Nachweisreagenz und (nichtmutiertem oder nichtmutiertem und mutiertem) Leptin darstellt.

Je kleiner der $K_D$-Wert ist, umso größer ist die Bindungsaffinität.

Als Bindungswert kann auch ein Fluoreszenzsignal verwendet werden.

Vorzugsweise wird der Bindungswert bei der Erfindung als Konzentration ausgedrückt, so dass mithin Konzentrationen miteinander verglichen werden.

Erfindungsgemäß ist wesentlich, dass der Bindungswert, d.h. der jeweils bestimmte Messwert, für die Bindung von nichtmutiertem Leptin an das erste Nachweisreagenz sowie von nichtmutiertem Leptin und mutiertem Leptin an das zweite Nachweisreagenz unter jeweils identischen Messbedingungen bestimmt wird.

Mithin ist die jeweilige Messung unter identischen Temperaturbedingungen, beispielsweise 20°C oder 25°C, unter identischen Pufferbedingungen, beispielsweise Phosphatgepufferte Salzlösung pH 7,5, mit identischen Verdünnungen für einen identischen Zeitraum, beispielsweise 2 Stunden, durchzuführen.

Das erfindungsgemäße Verfahren zum Nachweis von mutiertem Leptin wird in vitro unter Verwendung von isoliertem Probenmaterial durchgeführt. Mithin wird das erfindungsgemäße Verfahren nicht in vivo durchgeführt.

Gemäß einer bevorzugten Weiterbildung der Erfindung ist das erste Nachweisreagenz ein polyklonaler Antikörper, monoklonaler Antikörper oder ein Leptinrezeptor, der nichtmutiertes Leptin, jedoch mutiertes Leptin nicht oder mit maximal 50% des Bindungswerts von nichtmutiertem Leptin, bindet.

Gemäß einer weiteren bevorzugten Ausführungsform der Erfindung ist das zweite Nachweisreagenz ein polyklonaler oder monoklonaler Antikörper, wobei der polyklonale oder monoklonale Antikörper sowohl mutiertes als auch nichtmutiertes Leptin bindet.

Gemäß einer bevorzugten Variante ist das zweite Nachweisreagenz ein polyklonaler Antikörper, der sowohl mutiertes Leptin als auch nichtmutiertes Leptin bindet.

Polyklonale Antikörper können auf herkömmliche Weise hergestellt werden. Beispielsweise können polyklonale Antikörper gegen humanes Leptin hergestellt werden, indem Tiere mit Leptin, optional mit Adjuvantien, immunisiert werden. Als Tiere können beispielsweise Ziege, Kaninchen, Maus oder Ratte verwendet werden. Die von den verschiedenen B-Zellen produzierten Antikörper erkennen eine Vielzahl von Epitopen auf dem Leptin, so dass sowohl mutiertes als auch nichtmutiertes Leptin von den polyklonalen Antikörpern erkannt werden.

Alternativ können auch monoklonale Antikörper verwendet werden.

Auf herkömmliche Weise werden die nach Immunisierung mit Leptin erhaltenen B-Zellen mit einer Myelomzelle fusioniert (Hybridom-Technik).

Sodann werden die erhaltenen Hybridome vereinzelt und im Hinblick auf die von den Hybridom-Zellen produzierten Antikörper in Bezug auf deren Spezifität selektiert. Auf diese Weise können monoklonale Antikörper isoliert werden, die sowohl mutiertes als auch nichtmutiertes Leptin erkennen. Die Herstellung von monoklonalen Antikörpern und die Selektionierung von Hybridomzellen auf die Spezifität der Antikörper ist dem Fachmann bekannt.

Gemäß einer weiteren bevorzugten Ausführungsform der Erfindung umfasst der Leptinrezeptor die Leptinbindungsdomäne des humanen Leptinrezeptors, vorzugsweise der Isoform A, Isoform B, Isoform C, Isoform D oder Isoform E.

Der Leptinrezeptor ist ein Membran-gebundener Rezeptor, der eine extrazelluläre Leptinbindungsdomäne aufweist. Die extrazelluläre Leptinbindungdomäne ist über eine Transmembranregion mit einer zytoplasmatischen Domäne verbunden.

Es existieren beim Menschen fünf Isoformen, die Isoform A, Isoform B, Isoform C, Isoform D sowie Isoform E, die alternative Spleißvarianten darstellen.

Bei der Isoform B handelt es sich um die längste Variante des Leptinrezeptors.

Die Isoform A, Isoform C sowie die Isoform D des humanen Leptinrezeptors unterscheiden sich im Hinblick auf die zytoplasmatischen Domänen. Die zytoplasmatischen Domänen sind mit der Januskinase (JAK) verbunden. Bei Bindung von Leptin an den Leptinrezeptor kommt es zur Aggregation von Rezeptorhomodimeren, wodurch eine Aktivierung der Januskinase bewirkt wird. Im Zuge der Aktivierung kommt es zur Phosphorylierung von zytoplasmatischen Transkriptionsfaktoren STAT, insbesondere von STAT-3. Die aktivierten Signaltransduktoren und Aktivatoren der Transkription (STAT) wandern zum Zellkern und bewirken die Transkription von Genen.

Die Isoformen A, C sowie D tragen nur unwesentlich zur Signaltranskription bei.

Bei der Isoform E handelt es sich um eine lösliche Form eine Leptinrezeptors, die vermutlich durch proteolytische Spaltung im Rahmen eines Rezeptor-Sheddings von der Zelloberfläche abgespalten wird.

Erfindungsgemäß wird gemäß einer bevorzugten Ausführungsform die Leptinbindungsdomäne oder die Isoform E des Leptinrezeptors als zweites Nachweisreagenz verwendet.

Die Leptinbindungsdomäne können auch als Fusionsproteine verwendet werden. So kann gemäß einer erfindungsgemäßen Variante die Leptinbindungsdomäne oder die Isoform E mit dem Fc-Teil eines Antikörpers fusioniert werden. Bei Fusionierung mit dem Fc-Teil eines Antikörpers können Homodimere der Leptinbindungsdomäne bereitgestellt werden. Diese Homodimere ähneln den bei der natürlichen Leptinbindung auf einer Zelle in vivo induzierten Homodimeren.

Gemäß einer bevorzugten Variante der Erfindung werden die Aminosäuren Thr20 bis Asp839 der Isoform B bei der Herstellung eines Fusionsproteins verwendet. Vorzugsweise werden die Aminosäuren Thr20 bis Asp839 der Isoform B mit dem Fc-Teil eines Antikörpers, vorzugsweise als Homdimer, fusioniert. Alternativ ist beispielsweise auch die Isoform E bei der Herstellung von Fusionsproteinen, beispielsweise mit dem Fc-Teil eines Antikörpers, optional als Homodimer, erfindungsgemäß geeignet.

Es hat sich überraschenderweise gezeigt, dass die Leptinbindungsdomäne des natürlichen Leptinrezeptors selektiv nichtmutiertes Leptin bindet.

Als zweites Nachweisreagenz können auch Leptinrezeptoren verwendet werden, deren Sequenz nicht identisch mit der des natürlichen Leptinrezeptors ist.

Erfindungsgemäß können mithin Leptinrezeptorvarianten des natürlichen Leptinrezeptors verwendet werden, bei denen Aminosäuren deletiert oder substituiert durch andere Aminosäuren sind, sofern nichtmutiertes Leptin nach wie vor spezifisch gebunden wird.

Gemäß einer bevorzugten Weiterbildung weist der verwendete Leptin-Rezeptor wenigstens eine Sequenzidentität von 80%, vorzugsweise von wenigstens 90%, weiter bevorzugt von wenigstens 95%, noch weiter bevorzugt von wenigstens 98%, bevorzugt von wenigstens 99%, bezogen auf die Sequenz des natürlichen Leptinrezeptors, auf.

Gemäß einer weiteren bevorzugten Ausbildung der Erfindung ist der Leptinrezeptor eine lösliche Isoform, die vorzugsweise die Aminosäuren Thr20 bis Asp839 der Isoform B des humanen Leptinrezeptors aufweist.

Gemäß einer bevorzugten Weiterbildung weist der verwendete Leptinrezeptor wenigstens eine Sequenzidentität von 80%, vorzugsweise von wenigstens 90%, weiter bevorzugt von wenigstens 95%, noch weiter bevorzugt von wenigstens 98%, noch weiter bevorzugt von wenigstens 99%, bezogen auf die Sequenz der löslichen Isoform des natürlichen Leptinrezeptors, die vorzugsweise die Aminosäuren Thr20 bis Asp839 der Isoform B aufweist, auf.

Gemäß einer weiter bevorzugten Ausführungsform der Erfindung weist das mutierte Leptin in der Aminosäuresequenz wenigstens eine der folgenden Aminosäurenaustausche D100Y, N103K und/oder L72S auf.

Bei einer bevorzugten Ausführungsform weist das mutierte Leptin in der Aminosäuresequenz den Aminosäureaustausch D100Y auf.

Bei einer bevorzugten Ausführungsform weist das mutierte Leptin in der Aminosäuresequenz den Aminosäureaustausch N103K auf.

Bei einer bevorzugten Ausführungsform weist das mutierte Leptin in der Aminosäuresequenz den Aminosäureaustausch L72S auf.

Es hat sich überraschend gezeigt, dass der natürliche Leptinrezeptor nichtmutiertes Leptin hochselektiv bindet.

So wird bereits bei einem Aminosäureaustausch, beispielsweise bei dem Protein D100Y, das mutierte Leptin nicht mehr von der natürlichen Bindungsdomäne des Leptinrezeptors gebunden.

Das erfindungsgemäße Verfahren zum Nachweis von mutiertem Leptin umfasst folgende Schritte:

- Bestimmen der Bindung von nichtmutiertem Leptin aus einem, vorzugsweise ersten, flüssigen Probenmaterial an ein erstes Nachweisreagenz unter Erhalt eines ersten Bindungswerts,
- Bestimmen der Bindung sowohl von mutiertem Leptin als auch von nichtmutiertem Leptin aus einem, vorzugsweise zweiten, flüssigen Probenmaterial an ein zweites Nachweisreagenz unter Erhalt eines zweiten Bindungswerts,

wobei mutiertes Leptin vorhanden ist, wenn der erste Bindungswert kleiner als der zweite Bindungswert ist.

Das vorzugsweise erste und das vorzugsweise zweite flüssige Probenmaterial sind vor der Durchführung des erfindungsgemäßen Verfahrens, vorzugsweise zusammen, von demselben Subjekt isoliert worden.

In Abhängigkeit von dem verwendeten Messverfahren kann die Bestimmung des ersten Bindungswertes und des zweiten Bindungswertes in einer identischen Messprobe oder separat in zwei Messproben durchgeführt werden. Im ersten Fall werden die Bindungswerte gemeinsam in einer Messprobe bestimmt, und im zweiten Fall wird das Probenmaterial in zwei Messproben aufgeteilt und separat, jedoch optional parallel, vermessen.

Bei dem Subjekt handelt es sich vorzugsweise um ein Säugetier, weiter vorzugsweise um einen Menschen.

Bei dem, vorzugsweise ersten und vorzugsweise zweiten, flüssigen Probenmaterial kann es sich beispielsweise um das Serum oder Plasma einer einem Subjekt, vorzugsweise einem Menschen, entnommen Blutprobe, handeln. Nach Abnahme des Blutes kann der zelluläre Teil des Blutes vor der Gerinnung oder nach der Gerinnung abgetrennt werden. Sofern die Abtrennung vor der Gerinnung erfolgt, wird Blutplasma erhalten, erfolgt die Abtrennung nach der Gerinnung wird Blutserum erhalten.

Erfindungsgemäß kann als, vorzugsweise erstes und vorzugsweise zweites, flüssiges Probenmaterial sowohl Blutplasma als auch Blutserum verwendet werden.

Gemäß einer bevorzugten Variante der Erfindung wird Blutserum verwendet.

Die Blutprobe kann nach Abtrennung der zellulären Bestandteile getrennt werden, um ein, vorzugsweise erstes und ein vorzugsweise zweites, flüssiges Probenmaterial zu erhalten.

Nach geeigneter Verdünnung, beispielsweise in einem Puffer, beispielsweise in Phosphat-gepufferter Salzlösung, Tris-gepufferter Salzlösung oder Natriumbicarbonat-Puffer, werden das so verdünnte, vorzugsweise erste und vorzugsweise zweite, flüssige Probenmaterial mit dem ersten bzw. dem zweiten Nachweisreagenz in Kontakt gebracht und sodann der jeweilige Bindungswert bestimmt.

Der für den Bindungswert stehende Messwert kann durch verschiedene Verfahren bestimmt werden, wobei beispielsweise nach Einstellung von Gleichgewichtsbedingungen, die Konzentration an freiem Nachweisreagenz, freiem Leptin und/oder die Konzentration des Komplexes aus Nachweisreagenz und Leptin, bestimmt werden kann.

Beispielsweise kann ein Bindungswert mittels Gleichgewichtsdialyse, Ultrafiltration, Chromatographie, Elektrophorese, Zentrifugation oder biomolekulare Interaktionsanalyse (BIA) bestimmt werden.

Der Bindungswert, der ein Maß für die Affinität darstellt, kann auch über einen Kompetitionsassay bestimmt werden. Der Kompetitonsassay kann beispielsweise in Form eines RIA, ELISA, FIA oder LIA auf herkömmliche Weise durchgeführt werden.

Erfindungsgemäß ist wesentlich, dass bei der Bestimmung des Bindungswertes als Maß für die Affinität sowohl bei der Bestimmung des ersten Bindungswertes als auch bei der Bestimmung des zweiten Bindungswertes jeweils ein identisches Messverfahren unter identischen Bedingungen durchgeführt wird.

Gemäß einer weiteren bevorzugten Ausbildungsform des erfindungsgemäßen Verfahrens erfolgt die Bestimmung des ersten Bindungswertes und des zweiten Bindungswertes in einer einzigen Messprobe.

Bei dieser Weiterbildung der Erfindung ist keine Trennung in ein erstes und ein zweites flüssiges Probenmaterial erforderlich. Vielmehr erfolgt die Bestimmung des ersten Bindungswertes unter Verwendung des ersten Nachweisreagenzes und die Bestimmung des zweiten Bindungswertes unter Verwendung des zweiten Nachweisreagenzes in einer Messprobe, beispielsweise in einem Messvolumen. Die Messung der beiden Bindungswertes erfolgt dabei vorzugsweise gleichzeitig oder nahezu gleichzeitig.

Als Messverfahren eignen sich solche, bei denen das erste und das zweite Nachweisreagenz an partikuläre Festphasen gekoppelt sind. Diese partikulären Festphasen, an die jeweils das erste bzw. zweite Nachweisreagenz gekoppelt ist, sind anhand von unterschiedlichen Fluoreszenzeigenschaften, beispielsweise aufgrund der Verwendung unterschiedlichen Fluoreszenzfarbstoffe, unterscheidbar und können gemeinsam, vorzugsweise zeitgleich, beispielsweise mittels Durchflusszytometrie, vermessen werden. Diese Partikel-basierten Assays sind dem Fachmann beispielsweise als "Bead-based Multiplex-Assays" bekannt. Die Messung unter Verwendung von Partikel-basierten Multiplexverfahren können auch unter Verwendung von Biochips durchgeführt werden.

Wenn der erste Bindungswert geringer ist als der zweite Bindungswert, dann ist dies ein Nachweis dafür, dass das Probenmaterial mutiertes Leptin enthält. Der Anteil an mutiertem Leptin ist sodann ein Maß für den Anteil an physiologisch nicht aktivem Leptin.

Bei der Verwendung des ersten Nachweisreagenzes wird die Bindung von nichtmutiertem Leptin gemessen. Bei der Bestimmung des zweiten Bindungswertes wird unter Verwendung des zweiten Nachweisreagenzes sowohl die Bindung von mutiertem Leptin als auch von nichtmutiertem Leptin gemessen.

Wenn das aus demselben Subjekt gewonnene, vorzugsweise erste und vorzugsweise zweite, flüssige Probenmaterial kein mutiertes Leptin enthält, dann ist der mit dem ersten Nachweisreagenz enthaltene erste Bindungswert identisch oder nahezu identisch zu dem unter Verwendung des zweiten Nachweisreagenzes erhaltenen zweiten Bindungswert. Als nahezu identisch wird verstanden, dass der erste und der zweite Bindungswert sich um vorzugsweise bis zu weniger als 20 %, weiter vorzugsweise um bis zu weniger als 15 %, unterscheiden.

Vorzugsweise wird erfindungsgemäß als Verfahren ein ELISA, RIA, FIA, LIA, äußerst vorzugsweise ein ELISA, verwendet.

Gemäß einer weiteren bevorzugten Variante der Erfindung beträgt ein aus dem ersten und zweiten Bindungswert gebildeter Quotient Q:

Q = (erster Bindungswert/zweiter Bindungswert) 0,8 oder weniger

Weiter bevorzugt beträgt der Quotient Q 0,7 oder weniger, 0,6 oder weniger, 0,5 oder weniger, 0,4 oder weniger, 0,3 oder weniger, 0,2 oder weniger oder 0,1 oder weniger.

Bei einem Wert von 0,1 oder weniger bzw. einen gegen 0 gehenden Wert ist dies ein Indiz dafür, dass der Patient homozygot bezüglich der Leptinmutation ist. Sofern der Quotient zwischen 0,8 und 0,1, weiter vorzugsweise zwischen 0,6 und 0,3, liegt, dann ist dies ein Indiz dafür, dass der Patient heterozygot in Bezug auf die Leptinmutation ist. In diesem Fall sekretiert der Patient sowohl nichtmutiertes als auch mutiertes Leptin.

Das Ergebnis des erfindungsgemäßen Verfahrens erlaubt eine Differenzialdiagnose bei Fettleibigkeit.

Sofern ein erheblicher Anteil an mutiertem Leptin vorliegt, ist dies im Rahmen einer Differenzialdiagnose für den Mediziner von großer Bedeutung. In diesem Fall kann, obgleich der Patient einen gegebenenfalls stark erhöhten Pegel an Leptin, wobei dieses Leptin sowohl mutiertes als auch nichtmutiertes Leptin umfasst, hat, die Verabreichung eines Leptinersatzstoffes, beispielsweise Metreleptin, angezeigt sein, um die Fettleibigkeit und gegebenenfalls weitere physiologische Störungen zu therapieren.

Die vorliegende Erfindung erlaubt mithin eine Differenzialdiagnose, die im Falle von Patienten, die nur oder auch mutiertes Leptin synthetisieren, einen neuen Therapieansatz ermöglicht.

In Abhängigkeit von dem aus dem ersten Bindungswert und zweiten Bindungswert gebildeten Quotienten Q kann des Weiteren eine Diagnose über die voraussichtliche Menge an zu verabreichendem Leptinersatzstoff, beispielsweise Metreleptin, getroffen werden.

Für die von Fettleibigkeit betroffenen Patienten, insbesondere von Kindern und Jugendlichen, stellt die vorliegende Erfindung einen wesentlichen Fortschritt bei der Diagnose, insbesondere Differenzialdiagnose, und Therapie bei Fettleibigkeit dar.

Gemäß einer weiteren bevorzugten Ausführungsform des erfindungsgemäßen Verfahrens wird als das erste Nachweisreagenz ein polyklonaler Antikörper, monoklonaler Antikörper oder ein Leptinrezeptor, der nichtmutiertes Leptin, jedoch mutiertes Leptin nicht oder mit maximal 50% des Bindungswerts von nichtmutiertem Leptin, bindet, verwendet.

Gemäß einer weiteren bevorzugten Ausführungsform des erfindungsgemäßen Verfahrens wird als das zweite Nachweisreagenz ein polyklonaler oder monoklonaler Antikörper, wobei der polyklonale oder monoklonale Antikörper sowohl mutiertes Leptin als auch nichtmutiertes Leptin bindet, verwendet.

Gemäß einer weiteren bevorzugten Ausführungsform des erfindungsgemäßen Verfahrens wird bevorzugt, dass der Leptinrezeptor die Leptinbindungsdomäne des humanen Leptinrezeptors, vorzugsweise der Isoform A, Isoform B, Isoform D, umfasst.

Gemäß einer weiteren bevorzugten Weiterbildung des erfindungsgemäßen Verfahrens ist der Leptinrezeptor eine lösliche Isoform und weist vorzugsweise die Aminosäuren Thr20 bis Asp839 auf.

Weiterhin ist bevorzugt, dass das mutierte Leptin in der Aminosäuresequenz wenigstens einen der folgenden Aminosäureaustausche an den Sequenzpositionen D100Y, N103K und/oder L72S aufweist. Die Sequenzangaben beziehen sich dabei auf die Leptinsequenz einschließlich Signalpeptid.

Vorzugsweise ist das Verfahren ein immunologisches Verfahren, weiter vorzugsweise ein ELISA, RIA, FIA oder LIA.

Die oben im Hinblick auf die erfindungsgemäße Zusammenstellung von Nachweisreagenzien gemachten Ausführungen gelten entsprechend für das erfindungsgemäße Verfahren.

Die vorliegende Erfindung betrifft ferner die Verwendung eines Nachweisreagenzes, das nichtmutiertes Leptin mit einem ersten Bindungswert bindet, jedoch mutiertes Leptin nicht oder mit maximal 50% des Bindungswertes von nichtmutiertem Leptin bindet, bei der Diagnose von Fettleibigkeit.

Vorzugsweise wird das Nachweisreagenz bei der Differenzialdiagnose von Fettleibigkeit verwendet.

Bei dem Nachweisreagenz handelt es sich vorzugsweise um einen polyklonalen Antikörper, monoklonalen Antikörper oder einen Leptinrezeptor.

Gemäß einer weiteren bevorzugten Ausführungsform der erfindungsgemäßen Verwendung wird das Nachweisreagenz, im folgenden als erstes Nachweisreagenz bezeichnet, zusammen mit einem zweiten Nachweisreagenz verwendet. Das zweite Nachweisreagenz bindet sowohl mutiertes als auch nichtmutiertes Leptin mit einem zweiten Bindungswert.

Gemäß einer bevorzugten Weiterbildung der erfindungsgemäßen Verwendung ist das zweite Nachweisreagenz ein polyklonaler oder monoklonaler Antikörper, wobei der polyklonale oder monoklonale Antikörper sowohl mutiertes Leptin als auch nichtmutiertes Leptin bindet.

Erfindungsgemäß ist weiterhin bevorzugt, dass der als erstes Nachweisreagenz verwendete Leptinrezeptor die Leptinbindungsdomäne des humanen Leptinrezeptors, vorzugsweise der Isoform A, Isoform B, Isoform D, umfasst.

Gemäß einer weiteren Weiterbildung der erfindungsgemäßen Verwendung ist der als erstes Nachweisreagenz verwendete Leptinrezeptor eine lösliche Isoform und weist vorzugsweise die Aminosäuren Thr20 bis Asp839 der Isoform B auf.

Weiterhin ist bevorzugt, dass das mutierte Leptin in der Aminosäuresequenz wenigstens einen der folgenden Aminosäureaustausche an den Sequenzpositionen D100Y, N103K und/oder L72S aufweist, wobei sich die Sequenznummerierung auf die Aminosäuresequenz einschließlich Signalpeptide bezieht.

Gemäß einer bevorzugten Weiterbildung der erfindungsgemäßen Verwendung weist das mutierte Leptin einen Aminosäureaustausch D100Y in der Sequenz des natürlichen Leptins auf.

Gemäß einer bevorzugten Weiterbildung der erfindungsgemäßen Verwendung weist das mutierte Leptin einen Aminosäureaustausch N103K in der Sequenz des natürlichen Leptins auf.

Gemäß einer bevorzugten Weiterbildung der erfindungsgemäßen Verwendung weist das mutierte Leptin einen Aminosäureaustausch L72S in der Sequenz des natürlichen Leptins auf.

Vorzugsweise ist das Verfahren ein immunologisches Verfahren, weiter vorzugsweise eine ELISA, RIA, FIA oder LIA.

Die zu der erfindungsgemäßen Zusammenstellung von Nachweisreagenzien gemachten Ausführungen gelten für die erfindungsgemäße Verwendung entsprechend.

Die nachfolgenden Figuren und Beispiele dienen der Veranschaulichung der Erfindung, ohne jedoch die Erfindung zu beschränken.

**Figuren**

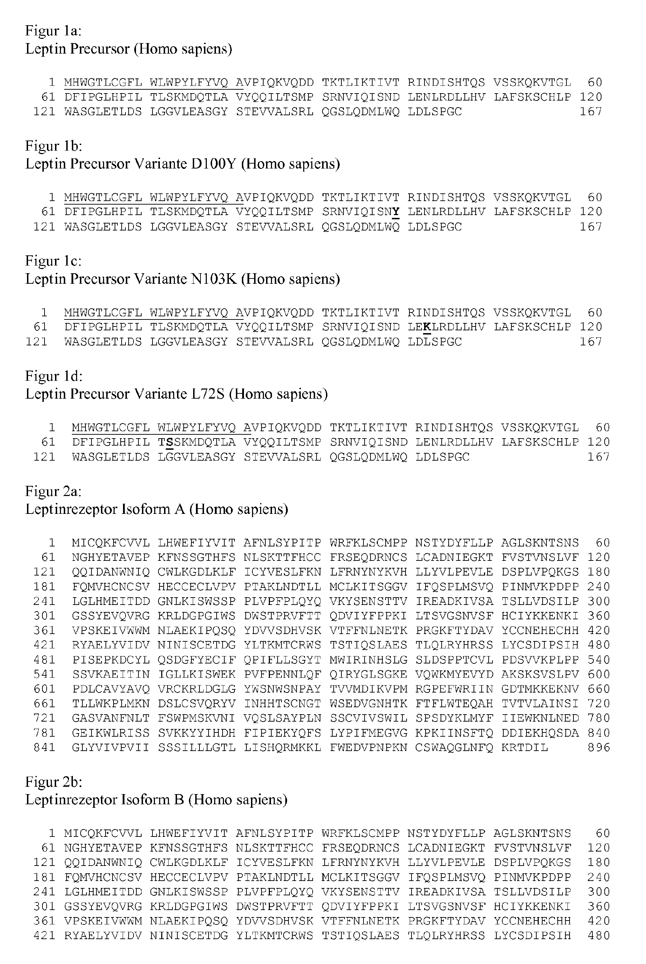

**Figur 1a** zeigt die humane Leptinvorläufersequenz, die das reife Leptin (Aminosäuren 22 bis 167) sowie das N-terminal angeordnete Signalpeptid (Aminosäuren 1 bis 21) umfasst.

**Figur 1b** zeigt die humane Leptinvorläufersequenz eines mutierten Leptins, das an der Aminosäureposition 100 einen Austausch von Asparaginsäure (D) gegen Tyrosin (Y) enthält.

**Figur 1c** zeigt die humane Leptinvorläufersequenz eines mutierten Leptins, das an der Aminosäureposition 103 einen Austausch von Asparagin (N) gegen Lysin (K) enthält.

**Figur 1d** zeigt die humane Leptinvorläufersequenz eines mutierten Leptins, das an der Aminosäureposition 72 einen Austausch von Leucin (L) gegen Serin (S) enthält.

**Figur 2a** zeigt die humane Leptinrezeptorsequenz der Isoform A.

**Figur 2b** zeigt die humane Leptinrezeptorsequenz der Isoform B.

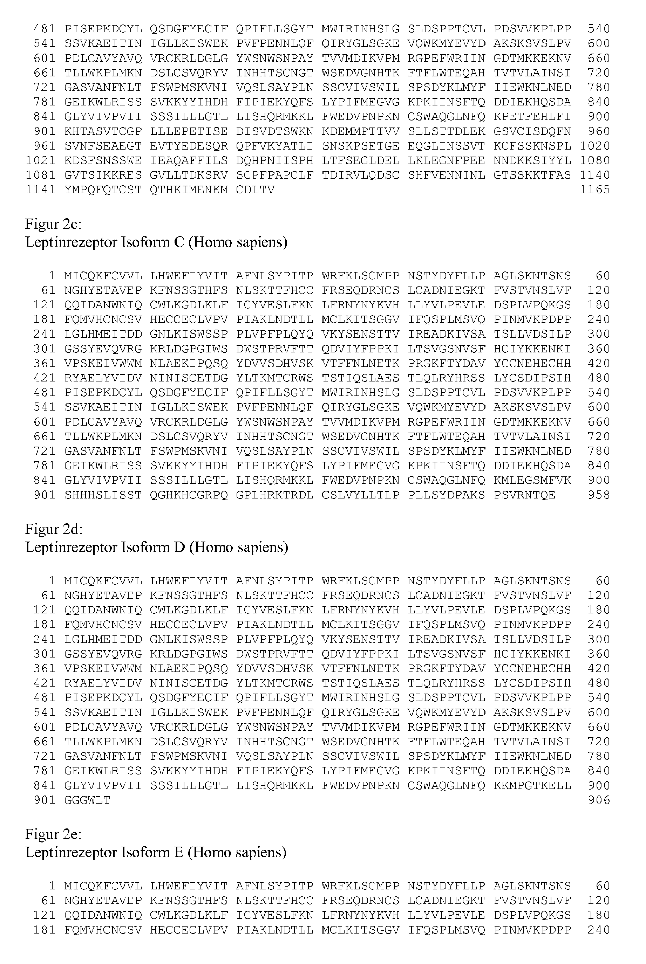

**Figur 2c** zeigt die humane Leptinrezeptorsequenz der Isoform C.

**Figur 2d** zeigt die humane Leptinrezeptorsequenz der Isoform D.

**Figur 2e** zeigt die humane Leptinrezeptorsequenz der Isoform E.

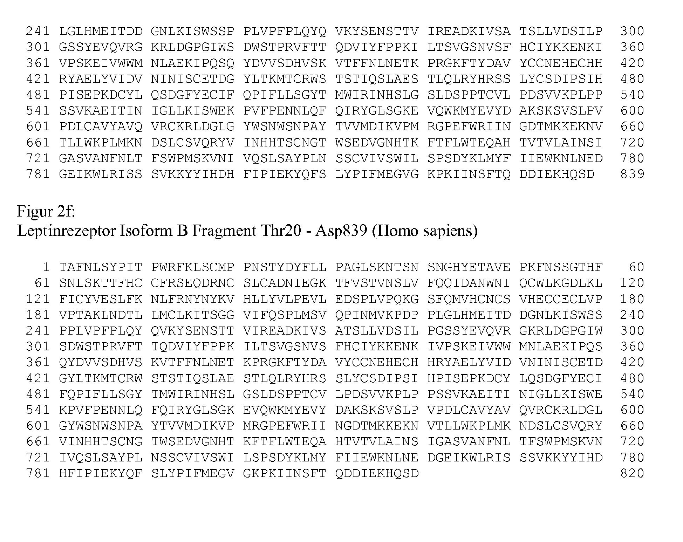

**Figur 2f** zeigt das Fragment Thr20 bis Asp839 der humanen Leptinrezeptorsequenz der Isoform B.

**Beispiele**

**Beispiel 1a: In-vitro Nachweis von Gesamt-Leptin**

Zur Herstellung eines Gesamt-Leptin ELISAs wurden handelsübliche Polystyrol Mikrotiterplatten verwendet (Fa. Greiner Bio-One GmbH, Frickenhausen, DE - Typ C8, Flat Bottom, Nr. 705071).

Adsorptiv wurde an diese Polystyrol Mikrotiterplatten als feste Phase ein kommerziell erhältlicher monoklonaler Maus-anti-humanes Leptin-IgG1 Erstantikörper in einer Konzentration von 2 μg/mL in phosphatgepufferter Salzlösung (PBS) bei pH 7,4 gebunden.

Die Platten wurden dazu über Nacht in einer feuchten Kammer gekühlt (2°C bis 8°C) inkubiert. Anschließend wurde die Antikörperlösung abgesaugt, und nicht gesättigte Bindungsstellen auf der Polystyroloberfläche mit Rinderserumalbumin (RSA) in 1 %iger Lösung in PBS über 4 Stunden bei pH 7,4 abgesättigt. Die Lösung wurde abgesaugt und die Platten direkt verwendet oder bis zur Verwendung getrocknet gelagert.

Die Messungen wurden in Doppelbestimmung durchgeführt. Alle Inkubationen wurden bei Raumtemperatur (20 °C bis 25°C) durchgeführt.

1. In alle benötigten Vertiefungen der Mikrotiterplatten wurden 100 µl Verdünnungspuffer (50 mM PBS-Puffer pH 7,4 mit 0,5 % RSA und 0,05 % Detergenz Tween-20) vorgelegt.

2. In die ersten 2 Vertiefungen wurden 20 µl Verdünnungspuffer pipettiert (Leerwert). Im Anschluss daran wurden jeweils 20 µl Leptin-Standard oder 20 µl der zu messenden Probe gegeben.

3. Die Vertiefungen der Platte wurden mit Klebefolie abgedeckt und 1 Stunde bei 200 bis 350 Upm geschüttelt.

4. Nach Ablauf der Inkubationszeit wurden die Lösungen abgesaugt und die Platte mit 300 µl Waschpuffer WP (50 mM PBS-Puffer pH 7,4 mit 0,5 % RSA) / Vertiefung fünfmal gewaschen. Der Waschpuffer verblieb vor dem Absaugen etwa 15 Sekunden in den Vertiefungen.

5. Im Anschluss an den letzten Waschschritt wurden 100 µl eines Meerettich-Peroxidase-(POD)-Konjugates eines anti-Maus-IgG1-Antikörpers in jede Vertiefung pipettiert und der Ansatz 30 Minuten bei 200 bis 350 Upm geschüttelt.

6. Nach Abschluss dieser Inkubation wurde die Platte wie in Schritt 4) beschrieben 5x gewaschen.

7. In jede Vertiefung wurden 100 µl der Substratlösung, stabilisiertes $H_2O_2$-Tetramethylbenzidin, pipettiert.

8. Die Platte wurde 15 Minuten im Dunkeln inkubiert, da das Substrat lichtempfindlich ist.

9. Nach Ablauf der Inkubationszeit wurde in jede Vertiefung 100 µl 0,1 M Schwefelsäure pipettiert.

10. Die Messung der Farbreaktion erfolgte innerhalb von 30 Minuten in einem Photometer für Mikrotiterplatten ("ELISA-Reader") bei 450 nm (Referenzfilter ≥590 nm).

Proben, die höhere Extinktionen als der Konzentrations-höchste Standard erzielten, lagen außerhalb der Standardkurve. Zur sicheren Bestimmung wurden diese Proben in einer zweiten Testdurchführung bei höherer Verdünnung nochmals gemessen.

Als Standards des ELISA wurde rekombinantes Leptin (Mediagnost, Reutlingen, DE) in einer Konzentrationen von 1, 10, 25, 50 und 100 ng/ml verwendet.

Von den Mittelwerten der optischen Dichte (OD) der Standardkonzentrationen und der Proben wurde der mittlere Leerwert OD abgezogen.

Die Standardkonzentrationen (x-Achse) wurden gegen die gemessene optische Dichte (y-Achse) aufgetragen und eine Standardkurve erstellt.

Aus der Standardkurve erhält man die Leptin-Konzentration der Kontrollen bzw. der Proben.

**Beispiel 1b: In-vitro Nachweis von funktionalem Leptin**

Für den Nachweis von funktionalem Leptin wurde der Testaufbau derart variiert, das an Polystyrol Mikrotiterplatten als feste Phase ein Leptinrezeptor gebunden wurde, der nichtmutiertes Leptin, jedoch mutiertes Leptin nicht oder maximal mit 50 % des Bindungswerts von nichtmutiertem Leptin bindet.

Als Leptinrezeptor wurde ein kommerziell erhältliches rekombinant hergestelltes Molekül mit der Aminosäuresequenz Thr20-Asp839 des humanen LeptinRezeptors verwendet (Recombinant Human Leptin R Fc Chimera, Catalog Number 389-LR/CF, R & D Systems, Minneapolis, MN, USA).

Der Leptinrezeptor wurde in einer Konzentration von 1,5 µg/mL in 10 mM Natrium-Carbonatpuffer bei pH 9,6 über Nacht in einer feuchten Kammer in gekühlter Umgebung (2°C bis 8°C) adsorptiv an Polystyrol Mikrotiterplatten gebunden.

Anschließend wurde die Beschichtungslösung abgesaugt, und nicht gesättigte Bindungsstellen auf der Polystyroloberfläche mit Rinderserumalbumin in 1 %iger Lösung in PBS über 4 Stunden bei pH 7,4 abgesättigt.

Die Lösung wurde abgesaugt und die Platten direkt verwendet oder getrocknet gelagert bis zur Verwendung. Die Messungen wurden in Doppelbestimmung durchgeführt. Alle Inkubationen wurden bei Raumtemperatur (25 °C) durchgeführt.

Die zu messenden Proben bzw. Kontrollen wurden mit Verdünnungspuffer (50 mM PBS-Puffer pH 7,4 mit 0,5 % RSA und 0,05 % Detergenz Tween-20) im Verhältnis 1:10 verdünnt.

1. In alle benötigten Vertiefungen der Mikrotiterplatten wurden 100 $\mu$l Standard, bzw. 100 $\mu$l vorverdünnte Kontroll- oder Probenlösung pipettiert.

2. Die Vertiefungen der Platte wurden mit Klebefolie abgedeckt und 2 Stunden bei 350 Upm geschüttelt.

3. Nach Ablauf der Inkubationszeit wurden die Lösungen abgesaugt und die Platte mit 300 $\mu$l Waschpuffer WP / Vertiefung dreimal gewaschen. Der Waschpuffer verblieb vor dem Absaugen etwa 15 Sekunden in den Vertiefungen.

4. Im Anschluss an den letzten Waschschritt wurden 100 $\mu$l eines Biotin-Konjugates eines kommerziell erhältlichen monoklonalen Maus-anti-humanes Leptin-IgG1 Antikörpers in jede Vertiefung pipettiert und der Ansatz 30 Minuten bei 350 Upm geschüttelt.

5. Nach Abschluss dieser Inkubation wurde die Platte, wie in Schritt 3 beschrieben, dreimal gewaschen.

6. Im Anschluss an den letzten Waschschritt werden z.B. 100 $\mu$l eines Streptavin-Meerrettichperoxidase-Konjugates in jede Vertiefung pipettiert und der Ansatz 30 Minuten bei 350 Upm geschüttelt.
Nach Abschluss dieser Inkubation wurde die Platte wie in Schritt 3) beschrieben 3x gewaschen.

7. In jede Vertiefung wurden 100 $\mu$l der Substratlösung, stabilisiertes $H_2O_2$-Tetramethylbenzidin, pipettiert.

8. Die Platte wurde 15 Minuten im Dunkeln bei 20 °C bis 25°C inkubiert, da das Substrat lichtempfindlich ist.

9. Nach Ablauf der Inkubationszeit wurde in jede Vertiefung 100 $\mu$l 0,1 M Schwefelsäure pipettiert.

10. Die Messung der Farbreaktion erfolgte innerhalb von 30 Minuten in einem Photometer für Mikrotiterplatten ("ELISA-Reader") bei 450 nm (Referenzfilter $\geq$590 nm).

Proben, die höhere Extinktionen als der Konzentrations-höchste Standard erzielten, lagen außerhalb der Standardkurve. Zur sicheren Bestimmung wurden diese Proben in einer zweiten Testdurchführung bei höherer Verdünnung nochmals gemessen.

Als Standards des ELISA wurde rekombinantes Leptin (Mediagnost, Reutlingen, DE) in einer Konzentrationen von 0, 0,2, 1, 2,5, 5, 7,5 und 10ng/ml verwendet.

Von den Mittelwerten der optischen Dichte (OD) der Standardkonzentrationen und der Proben wurde der mittlere Leerwert OD abgezogen.

Die Standardkonzentrationen (x-Achse) wurden gegen die gemessene optische Dichte (y-Achse) aufgetragen und eine Standardkurve erstellt.

Aus der Standardkurve erhält man die Leptin-Konzentration der verdünnten Kontrollen bzw. der Proben, und nach Multiplikation mit dem Verdünnungsfaktor dann die Leptin-Konzentration der unverdünnten Kontrollen bzw. Proben.

Nicht mutiertes funktionales Leptin wurde in diesem Test in einer Konzentration gemessen, die der im Leptin ELISA für Gesamt-Leptin gemessenen Konzentration entspricht.

Beispiel 1c: Auswertung

Humane Leptin cDNA (NCBI Reference Sequence: NM_000230.2) wurde verwendet um Punktmutationen durch Site-directed-mutagenesis zu erzeugen.

Dazu wurde der Q5® Site-Directed Mutagenesis Kit der Fa. New England Biolabs GmbH (Frankfurt am Main, DE) gemäß Herstellerangaben verwendet.

HEK293 Zellen wurden mit humanem Leptin (pcDNA3.1+-leptin_wt) oder mutiertem Leptin (pcDNA3.1+-leptin_D100Y oder pcDNA3.1+-leptin_N103K), wie in Sambrook et al. (Molecular cloning: a laboratory handbook, 2nd edition, 1998, Corld Spring Harbor Laboratory Press, Corld Spring Harbor, USA) beschrieben, transient transfiziert.

48 h nach der Transfektion wurden die jeweiligen Zellen lysiert und 100 $\mu$l der Überstände als Proben 1 bis 3 in den in Beispiel 1a und Beispiel 1 b beschriebenen ELISA Tests verwendet.

Weiterhin wurden Serum-Proben von 5 Blutspendern sowie gereinigtes rekombinantes humanes Leptin sowie gereinigtes rekombinantes humanes Leptin Mutation D100Y verwendet.

Messergebnisse von verschiedenen Proben mit den in Beispiel 1a und Beispiel 1 b beschriebenen Tests sind in Tabelle 1 dargestellt:

**Tabelle 1: Vergleich Messwerte Gesamt gegen funktionales Leptin**

| Probe Nr.: | HEK293 transient exprimierte Zellkulturüberstände | | Messwerte funktionales Leptin | Messwerte Gesamt-Leptin |
|---|---|---|---|---|
| 1 | Leptin nicht mutiert | | 490,2 ng/mL | 577,7 ng/mL |
| 2 | Leptin Mutation D100Y | | < 0,5 ng/mL | 193,1 ng/mL |
| 3 | Leptin Mutation N103K | | < 0.35 ng/mL | 37,1 ng/mL |
| | **rekombinantes Leptin gereinigt** | | | |
| 4 | Leptin nicht mutiert | | 277,7 µg/mL | 335,2 µg/mL |
| 5 | Leptin Mutation D100Y | | 108 ng/mL | 8,03 µg/mL |
| | **Internationaler Standard der WHO für human Leptin WHO IS NIBSC 97/594** | | | |
| 6 | | nominal 2 ng/mL: | 1,83 ng/mL | 1,84 ng/mL |
| 7 | | nominal 6 ng/mL: | 6,05 ng/mL | 6,1 ng/mL |
| | **Serum- / Plasmaproben Blutspender** | | | |
| 8 | **CS-2** | | 2,81 ng/mL | 2,46 ng/mL |
| 9 | **DO-2226** | | 4,61 ng/mL | 5,05 ng/mL |
| 10 | IDB-883- | | 7,25 ng/mL | 8,31 ng/mL |
| 11 | **IDB-8960** | | 23,96 ng/mL | 25,05 ng/mL |
| 12 | **BB-3935** | | 34,12 ng/mL | 34,84 ng/mL |

**Patentansprüche**

1. Zusammenstellung von Nachweisreagenzien,
   **dadurch gekennzeichnet,**
   **dass** die Zusammenstellung ein erstes und ein zweites Nachweisreagenz umfasst,
   wobei das erste Nachweisreagenz nichtmutiertes Leptin mit einem ersten Bindungswert, jedoch mutiertes Leptin nicht oder maximal mit 50 % des Bindungswerts von nichtmutiertem Leptin bindet, und wobei das zweite Nachweisreagenz sowohl mutiertes als auch nichtmutiertes Leptin mit einem zweiten Bindungswert bindet.

2. Zusammenstellung nach Anspruch 1,
   **dadurch gekennzeichnet,**
   **dass** das erste Nachweisreagenz ein polyklonaler Antikörper, monoklonaler Antikörper oder ein Leptinrezeptor ist, der nichtmutiertes Leptin, jedoch mutiertes Leptin nicht oder maximal mit 50 % des Bindungswerts von nichtmutiertem Leptin bindet.

3. Zusammenstellung nach Anspruch 1 oder 2,
   **dadurch gekennzeichnet,**
   **dass** das zweite Nachweisreagenz ein polyklonaler oder monoklonaler Antikörper ist, wobei der polyklonale oder monoklonale Antikörper sowohl mutiertes Leptin als auch nichtmutiertes Leptin bindet.

4. Zusammenstellung nach Anspruch 2,
   **dadurch gekennzeichnet,**
   **dass** der Leptinrezeptor die Leptinbindungsdomäne des humanen Leptinrezeptors, vorzugsweise der Isoform A, Isoform B, Isoform C, Isoform D oder Isoform E, umfasst.

5. Zusammenstellung nach Anspruch 2 oder 4,
   **dadurch gekennzeichnet,**
   **dass** der Leptinrezeptor eine lösliche Isoform ist und vorzugsweise die Aminosäuren Thr20 bis Asp839 der Isoform B des humanen Leptinrezeptors aufweist.

6. Zusammenstellung nach einem der Ansprüche 1 bis 5,
   **dadurch gekennzeichnet,**
   **dass** das mutierte Leptin in der Aminosäuresequenz wenigsten einen der folgenden Aminosäureaustausche an den Sequenzpositionen D100Y, N103K und/oder L72S aufweist.

7. In-vitro-Verfahren zum Nachweis von mutiertem Leptin,
   **dadurch gekennzeichnet,**
   **dass** das Verfahren folgende Schritte umfasst:

   - Bestimmen der Bindung von nichtmutiertem Leptin aus einem, vorzugsweise ersten, flüssigen Probenmaterial an ein erstes Nachweisreagenz unter Erhalt eines ersten Bindungswerts,
   - Bestimmen der Bindung sowohl von mutiertem Leptin als auch von nichtmutiertem Leptin aus einem, vorzugsweise zweiten, flüssigen Probenmaterial an ein zweites Nachweisreagenz unter Erhalt eines zweiten Bindungswerts,

   wobei mutiertes Leptin vorhanden ist, wenn der erste Bindungswert kleiner als der zweite Bindungswert ist.

8. Verfahren nach Anspruch 7,
   **dadurch gekennzeichnet,**
   **dass** das erste Nachweisreagenz ein polyklonaler Antikörper, monoklonaler Antikörper oder ein Leptinrezeptor ist, der nichtmutiertes Leptin, jedoch mutiertes nicht Leptin oder maximal mit 50 % des Bindungswerts von nichtmutiertem Leptin bindet.

9. Verfahren nach einem der Ansprüche 7 oder 8,
   **dadurch gekennzeichnet,**
   **dass** das zweite Nachweisreagenz ein polyklonaler oder monoklonaler Antikörper ist, wobei der polyklonale oder monoklonale Antikörper sowohl mutiertes Leptin als auch nichtmutiertes Leptin bindet.

10. Verfahren nach Anspruch 8,
    **dadurch gekennzeichnet,**
    **dass** der Leptinrezeptor die Leptinbindungsdomäne des humanen Leptinrezeptor, vorzugsweise der Isoform A, Isoform B, Isoform C, Isoform D oder Isoform E, umfasst.

11. Verfahren nach Anspruch 8 oder 10,

**dadurch gekennzeichnet,**
**dass** der Leptinrezeptor eine lösliche Isoform ist und vorzugsweise die Aminosäuren Thr20 bis Asp839 der Isoform B des humanen Leptinrezeptors aufweist.

**12.** Verfahren nach einem der Ansprüche 7 bis 11,
**dadurch gekennzeichnet,**
**dass** das mutierte Leptin in der Aminosäuresequenz wenigstens einen der folgenden Aminosäureaustausche D100Y, N103K und/oder L72S aufweist.

**13.** Verfahren nach einem der Ansprüche 7 bis 12,
**dadurch gekennzeichnet,**
**dass** das Verfahren ein immunologisches Verfahren, vorzugsweise ein ELISA, RIA, FIA oder LIA, ist.

**14.** Verfahren nach einem der Ansprüche 7 bis 13,
**dadurch gekennzeichnet,**
**dass** ein aus dem ersten und dem zweiten Bindungswert gebildeter Quotient Q:

$$Q = (\text{erster Bindungswert})/(\text{zweiter Bindungswert})$$

0,8 oder weniger beträgt.

**15.** Verfahren nach einem der Ansprüche 7 bis 14,
**dadurch gekennzeichnet,**
**dass** das die Bestimmung des ersten Bindungswertes und des zweiten Bindungswertes in einer Messprobe erfolgt.

**16.** Verwendung eines Nachweisreagenzes, das nichtmutiertes Leptin mit einem ersten Bindungswert, jedoch mutiertes Leptin nicht oder maximal mit 50 % des Bindungswerts von nichtmutiertem Leptin bindet, bei der Diagnose von Fettleibigkeit.

Figur 1a:
Leptin Precursor (Homo sapiens)

```
  1 MHWGTLCGFL WLWPYLFYVQ AVPIQKVQDD TKTLIKTIVT RINDISHTQS VSSKQKVTGL  60
 61 DFIPGLHPIL TLSKMDQTLA VYQQILTSMP SRNVIQISND LENLRDLLHV LAFSKSCHLP 120
121 WASGLETLDS LGGVLEASGY STEVVALSRL QGSLQDMLWQ LDLSPGC            167
```

Figur 1b:
Leptin Precursor Variante D100Y (Homo sapiens)

```
  1 MHWGTLCGFL WLWPYLFYVQ AVPIQKVQDD TKTLIKTIVT RINDISHTQS VSSKQKVTGL  60
 61 DFIPGLHPIL TLSKMDQTLA VYQQILTSMP SRNVIQISN**Y** LENLRDLLHV LAFSKSCHLP 120
121 WASGLETLDS LGGVLEASGY STEVVALSRL QGSLQDMLWQ LDLSPGC            167
```

Figur 1c:
Leptin Precursor Variante N103K (Homo sapiens)

```
  1 MHWGTLCGFL WLWPYLFYVQ AVPIQKVQDD TKTLIKTIVT RINDISHTQS VSSKQKVTGL  60
 61 DFIPGLHPIL TLSKMDQTLA VYQQILTSMP SRNVIQISND LE**K**LRDLLHV LAFSKSCHLP 120
121 WASGLETLDS LGGVLEASGY STEVVALSRL QGSLQDMLWQ LDLSPGC            167
```

Figur 1d:
Leptin Precursor Variante L72S (Homo sapiens)

```
  1 MHWGTLCGFL WLWPYLFYVQ AVPIQKVQDD TKTLIKTIVT RINDISHTQS VSSKQKVTGL  60
 61 DFIPGLHPIL T**S**SKMDQTLA VYQQILTSMP SRNVIQISND LENLRDLLHV LAFSKSCHLP 120
121 WASGLETLDS LGGVLEASGY STEVVALSRL QGSLQDMLWQ LDLSPGC            167
```

Figur 2a:
Leptinrezeptor Isoform A (Homo sapiens)

```
  1 MICQKFCVVL LHWEFIYVIT AFNLSYPITP WRFKLSCMPP NSTYDYFLLP AGLSKNTSNS  60
 61 NGHYETAVEP KFNSSGTHFS NLSKTTFHCC FRSEQDRNCS LCADNIEGKT FVSTVNSLVF 120
121 QQIDANWNIQ CWLKGDLKLF ICYVESLFKN LFRNYNYKVH LLYVLPEVLE DSPLVPQKGS 180
181 FQMVHCNCSV HECCECLVPV PTAKLNDTLL MCLKITSGGV IFQSPLMSVQ PINMVKPDPP 240
241 LGLHMEITDD GNLKISWSSP PLVPFPLQYQ VKYSENSTTV IREADKIVSA TSLLVDSILP 300
301 GSSYEVQVRG KRLDGPGIWS DWSTPRVFTT QDVIYFPPKI LTSVGSNVSF HCIYKKENKI 360
361 VPSKEIVWWM NLAEKIPQSQ YDVVSDHVSK VTFFNLNETK PRGKFTYDAV YCCNEHECHH 420
421 RYAELYVIDV NINISCETDG YLTKMTCRWS TSTIQSLAES TLQLRYHRSS LYCSDIPSIH 480
481 PISEPKDCYL QSDGFYECIF QPIFLLSGYT MWIRINHSLG SLDSPPTCVL PDSVVKPLPP 540
541 SSVKAEITIN IGLLKISWEK PVFPENNLQF QIRYGLSGKE VQWKMYEVYD AKSKSVSLPV 600
601 PDLCAVYAVQ VRCKRLDGLG YWSNWSNPAY TVVMDIKVPM RGPEFWRIIN GDTMKKEKNV 660
661 TLLWKPLMKN DSLCSVQRYV INHHTSCNGT WSEDVGNHTK FTFLWTEQAH TVTVLAINSI 720
721 GASVANFNLT FSWPMSKVNI VQSLSAYPLN SSCVIVSWIL SPSDYKLMYF IIEWKNLNED 780
781 GEIKWLRISS SVKKYYIHDH FIPIEKYQFS LYPIFMEGVG KPKIINSFTQ DDIEKHQSDA 840
841 GLYVIVPVII SSSILLLGTL LISHQRMKKL FWEDVPNPKN CSWAQGLNFQ KRTDIL     896
```

Figur 2b:
Leptinrezeptor Isoform B (Homo sapiens)

```
  1 MICQKFCVVL LHWEFIYVIT AFNLSYPITP WRFKLSCMPP NSTYDYFLLP AGLSKNTSNS  60
 61 NGHYETAVEP KFNSSGTHFS NLSKTTFHCC FRSEQDRNCS LCADNIEGKT FVSTVNSLVF 120
121 QQIDANWNIQ CWLKGDLKLF ICYVESLFKN LFRNYNYKVH LLYVLPEVLE DSPLVPQKGS 180
181 FQMVHCNCSV HECCECLVPV PTAKLNDTLL MCLKITSGGV IFQSPLMSVQ PINMVKPDPP 240
241 LGLHMEITDD GNLKISWSSP PLVPFPLQYQ VKYSENSTTV IREADKIVSA TSLLVDSILP 300
301 GSSYEVQVRG KRLDGPGIWS DWSTPRVFTT QDVIYFPPKI LTSVGSNVSF HCIYKKENKI 360
361 VPSKEIVWWM NLAEKIPQSQ YDVVSDHVSK VTFFNLNETK PRGKFTYDAV YCCNEHECHH 420
421 RYAELYVIDV NINISCETDG YLTKMTCRWS TSTIQSLAES TLQLRYHRSS LYCSDIPSIH 480
```

```
481 PISEPKDCYL QSDGFYECIF QPIFLLSGYT MWIRINHSLG SLDSPPTCVL PDSVVKPLPP 540
541 SSVKAEITIN IGLLKISWEK PVFPENNLQF QIRYGLSGKE VQWKMYEVYD AKSKSVSLPV 600
601 PDLCAVYAVQ VRCKRLDGLG YWSNWSNPAY TVVMDIKVPM RGPEFWRIIN GDTMKKEKNV 660
661 TLLWKPLMKN DSLCSVQRYV INHHTSCNGT WSEDVGNHTK FTFLWTEQAH TVTVLAINSI 720
721 GASVANFNLT FSWPMSKVNI VQSLSAYPLN SSCVIVSWIL SPSDYKLMYF IIEWKNLNED 780
781 GEIKWLRISS SVKKYYIHDH FIPIEKYQFS LYPIFMEGVG KPKIINSFTQ DDIEKHQSDA 840
841 GLYVIVPVII SSSILLLGTL LISHQRMKKL FWEDVPNPKN CSWAQGLNFQ KPETFEHLFI 900
901 KHTASVTCGP LLLEPETISE DISVDTSWKN KDEMMPTTVV SLLSTTDLEK GSVCISDQFN 960
961 SVNFSEAEGT EVTYEDESQR QPFVKYATLI SNSKPSETGE EQGLINSSVT KCFSSKNSPL 1020
1021 KDSFSNSSWE IEAQAFFILS DQHPNIISPH LTFSEGLDEL LKLEGNFPEE NNDKKSIYYL 1080
1081 GVTSIKKRES GVLLTDKSRV SCPFPAPCLF TDIRVLQDSC SHFVENNINL GTSSKKTFAS 1140
1141 YMPQFQTCST QTHKIMENKM CDLTV                                      1165
```

Figur 2c:
Leptinrezeptor Isoform C (Homo sapiens)

```
  1 MICQKFCVVL LHWEFIYVIT AFNLSYPITP WRFKLSCMPP NSTYDYFLLP AGLSKNTSNS   60
 61 NGHYETAVEP KFNSSGTHFS NLSKTTFHCC FRSEQDRNCS LCADNIEGKT FVSTVNSLVF  120
121 QQIDANWNIQ CWLKGDLKLF ICYVESLFKN LFRNYNYKVH LLYVLPEVLE DSPLVPQKGS  180
181 FQMVHCNCSV HECCECLVPV PTAKLNDTLL MCLKITSGGV IFQSPLMSVQ PINMVKPDPP  240
241 LGLHMEITDD GNLKISWSSP PLVPFPLQYQ VKYSENSTTV IREADKIVSA TSLLVDSILP  300
301 GSSYEVQVRG KRLDGPGIWS DWSTPRVFTT QDVIYFPPKI LTSVGSNVSF HCIYKKENKI  360
361 VPSKEIVWWM NLAEKIPQSQ YDVVSDHVSK VTFFNLNETK PRGKFTYDAV YCCNEHECHH  420
421 RYAELYVIDV NINISCETDG YLTKMTCRWS TSTIQSLAES TLQLRYHRSS LYCSDIPSIH  480
481 PISEPKDCYL QSDGFYECIF QPIFLLSGYT MWIRINHSLG SLDSPPTCVL PDSVVKPLPP  540
541 SSVKAEITIN IGLLKISWEK PVFPENNLQF QIRYGLSGKE VQWKMYEVYD AKSKSVSLPV  600
601 PDLCAVYAVQ VRCKRLDGLG YWSNWSNPAY TVVMDIKVPM RGPEFWRIIN GDTMKKEKNV  660
661 TLLWKPLMKN DSLCSVQRYV INHHTSCNGT WSEDVGNHTK FTFLWTEQAH TVTVLAINSI  720
721 GASVANFNLT FSWPMSKVNI VQSLSAYPLN SSCVIVSWIL SPSDYKLMYF IIEWKNLNED  780
781 GEIKWLRISS SVKKYYIHDH FIPIEKYQFS LYPIFMEGVG KPKIINSFTQ DDIEKHQSDA  840
841 GLYVIVPVII SSSILLLGTL LISHQRMKKL FWEDVPNPKN CSWAQGLNFQ KMLEGSMFVK  900
901 SHHHSLISST QGHKHCGRPQ GPLHRKTRDL CSLVYLLTLP PLLSYDPAKS PSVRNTQE    958
```

Figur 2d:
Leptinrezeptor Isoform D (Homo sapiens)

```
  1 MICQKFCVVL LHWEFIYVIT AFNLSYPITP WRFKLSCMPP NSTYDYFLLP AGLSKNTSNS   60
 61 NGHYETAVEP KFNSSGTHFS NLSKTTFHCC FRSEQDRNCS LCADNIEGKT FVSTVNSLVF  120
121 QQIDANWNIQ CWLKGDLKLF ICYVESLFKN LFRNYNYKVH LLYVLPEVLE DSPLVPQKGS  180
181 FQMVHCNCSV HECCECLVPV PTAKLNDTLL MCLKITSGGV IFQSPLMSVQ PINMVKPDPP  240
241 LGLHMEITDD GNLKISWSSP PLVPFPLQYQ VKYSENSTTV IREADKIVSA TSLLVDSILP  300
301 GSSYEVQVRG KRLDGPGIWS DWSTPRVFTT QDVIYFPPKI LTSVGSNVSF HCIYKKENKI  360
361 VPSKEIVWWM NLAEKIPQSQ YDVVSDHVSK VTFFNLNETK PRGKFTYDAV YCCNEHECHH  420
421 RYAELYVIDV NINISCETDG YLTKMTCRWS TSTIQSLAES TLQLRYHRSS LYCSDIPSIH  480
481 PISEPKDCYL QSDGFYECIF QPIFLLSGYT MWIRINHSLG SLDSPPTCVL PDSVVKPLPP  540
541 SSVKAEITIN IGLLKISWEK PVFPENNLQF QIRYGLSGKE VQWKMYEVYD AKSKSVSLPV  600
601 PDLCAVYAVQ VRCKRLDGLG YWSNWSNPAY TVVMDIKVPM RGPEFWRIIN GDTMKKEKNV  660
661 TLLWKPLMKN DSLCSVQRYV INHHTSCNGT WSEDVGNHTK FTFLWTEQAH TVTVLAINSI  720
721 GASVANFNLT FSWPMSKVNI VQSLSAYPLN SSCVIVSWIL SPSDYKLMYF IIEWKNLNED  780
781 GEIKWLRISS SVKKYYIHDH FIPIEKYQFS LYPIFMEGVG KPKIINSFTQ DDIEKHQSDA  840
841 GLYVIVPVII SSSILLLGTL LISHQRMKKL FWEDVPNPKN CSWAQGLNFQ KKMPGTKELL  900
901 GGGWLT                                                            906
```

Figur 2e:
Leptinrezeptor Isoform E (Homo sapiens)

```
  1 MICQKFCVVL LHWEFIYVIT AFNLSYPITP WRFKLSCMPP NSTYDYFLLP AGLSKNTSNS   60
 61 NGHYETAVEP KFNSSGTHFS NLSKTTFHCC FRSEQDRNCS LCADNIEGKT FVSTVNSLVF  120
121 QQIDANWNIQ CWLKGDLKLF ICYVESLFKN LFRNYNYKVH LLYVLPEVLE DSPLVPQKGS  180
181 FQMVHCNCSV HECCECLVPV PTAKLNDTLL MCLKITSGGV IFQSPLMSVQ PINMVKPDPP  240
```

```
241 LGLHMEITDD GNLKISWSSP PLVPFPLQYQ VKYSENSTTV IREADKIVSA TSLLVDSILP 300
301 GSSYEVQVRG KRLDGPGIWS DWSTPRVFTT QDVIYFPPKI LTSVGSNVSF HCIYKKENKI 360
361 VPSKEIVWWM NLAEKIPQSQ YDVVSDHVSK VTFFNLNETK PRGKFTYDAV YCCNEHECHH 420
421 RYAELYVIDV NINISCETDG YLTKMTCRWS TSTIQSLAES TLQLRYHRSS LYCSDIPSIH 480
481 PISEPKDCYL QSDGFYECIF QPIFLLSGYT MWIRINHSLG SLDSPPTCVL PDSVVKPLPP 540
541 SSVKAEITIN IGLLKISWEK PVFPENNLQF QIRYGLSGKE VQWKMYEVYD AKSKSVSLPV 600
601 PDLCAVYAVQ VRCKRLDGLG YWSNWSNPAY TVVMDIKVPM RGPEFWRIIN GDTMKKEKNV 660
661 TLLWKPLMKN DSLCSVQRYV INHHTSCNGT WSEDVGNHTK FTFLWTEQAH TVTVLAINSI 720
721 GASVANFNLT FSWPMSKVNI VQSLSAYPLN SSCVIVSWIL SPSDYKLMYF IIEWKNLNED 780
781 GEIKWLRISS SVKKYYIHDH FIPIEKYQFS LYPIFMEGVG KPKIINSFTQ DDIEKHQSD  839
```

Figur 2f:
Leptinrezeptor Isoform B Fragment Thr20 - Asp839 (Homo sapiens)

```
  1 TAFNLSYPIT PWRFKLSCMP PNSTYDYFLL PAGLSKNTSN SNGHYETAVE PKFNSSGTHF  60
 61 SNLSKTTFHC CFRSEQDRNC SLCADNIEGK TFVSTVNSLV FQQIDANWNI QCWLKGDLKL 120
121 FICYVESLFK NLFRNYNYKV HLLYVLPEVL EDSPLVPQKG SFQMVHCNCS VHECCECLVP 180
181 VPTAKLNDTL LMCLKITSGG VIFQSPLMSV QPINMVKPDP PLGLHMEITD DGNLKISWSS 240
241 PPLVPFPLQY QVKYSENSTT VIREADKIVS ATSLLVDSIL PGSSYEVQVR GKRLDGPGIW 300
301 SDWSTPRVFT TQDVIYFPPK ILTSVGSNVS FHCIYKKENK IVPSKEIVWW MNLAEKIPQS 360
361 QYDVVSDHVS KVTFFNLNET KPRGKFTYDA VYCCNEHECH HRYAELYVID VNINISCETD 420
421 GYLTKMTCRW STSTIQSLAE STLQLRYHRS SLYCSDIPSI HPISEPKDCY LQSDGFYECI 480
481 FQPIFLLSGY TMWIRINHSL GSLDSPPTCV LPDSVVKPLP PSSVKAEITI NIGLLKISWE 540
541 KPVFPENNLQ FQIRYGLSGK EVQWKMYEVY DAKSKSVSLP VPDLCAVYAV QVRCKRLDGL 600
601 GYWSNWSNPA YTVVMDIKVP MRGPEFWRII NGDTMKKEKN VTLLWKPLMK NDSLCSVQRY 660
661 VINHHTSCNG TWSEDVGNHT KFTFLWTEQA HTVTVLAINS IGASVANFNL TFSWPMSKVN 720
721 IVQSLSAYPL NSSCVIVSWI LSPSDYKLMY FIIEWKNLNE DGEIKWLRIS SSVKKYYIHD 780
781 HFIPIEKYQF SLYPIFMEGV GKPKIINSFT QDDIEKHQSD                       820
```

Europäisches
Patentamt

European
Patent Office

Office européen
des brevets

**EUROPÄISCHER RECHERCHENBERICHT**

Nummer der Anmeldung

EP 15 15 9303

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | N. LAHLOU ET AL: "Soluble leptin receptor in serum of subjects with complete resistance to leptin: relation to fat mass", DIABETES, Bd. 49, Nr. 8, 1. August 2000 (2000-08-01), Seiten 1347-1352, XP055041870, ISSN: 0012-1797, DOI: 10.2337/diabetes.49.8.1347 * das ganze Dokument, insbesondere Seite 1348, Absatz "Western blot analysis of eluates" * | 1-6 | INV. G01N33/74 C07K14/575 |
| X | WU ZIDA ET AL: "Quantification of the soluble leptin receptor in human blood by ligand-mediated immunofunctional assay", JOURNAL OF CLINICAL ENDOCRINOLOGY & METABOLISM, Bd. 87, Nr. 6, 1. Juni 2002 (2002-06-01), Seiten 2931-2939, XP002323286, * das ganze Dokument, insbesondere Seite 2932, Absätze "Reagents and equipment", "Characterization and selection of the mAbs", "Assay standards and serum control samples" * | 1-6 | |
| A,D | MARTIN WABITSCH ET AL: "Biologically Inactive Leptin and Early-Onset Extreme Obesity", NEW ENGLAND JOURNAL OF MEDICINE, Bd. 372, Nr. 1, 1. Januar 2015 (2015-01-01), Seiten 48-54, XP055196767, ISSN: 0028-4793, DOI: 10.1056/NEJMoa1406653 * das ganze Dokument * | 1-16 | |

RECHERCHIERTE SACHGEBIETE (IPC)

G01N
C07K

-/--

Der vorliegende Recherchenbericht wurde für alle Patentansprüche erstellt

| Recherchenort | Abschlußdatum der Recherche | Prüfer |
|---|---|---|
| München | 23. Juni 2015 | Weber, Peter |

KATEGORIE DER GENANNTEN DOKUMENTE

X : von besonderer Bedeutung allein betrachtet
Y : von besonderer Bedeutung in Verbindung mit einer
      anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
      nach dem Anmeldedatum veröffentlicht worden ist
D : in der Anmeldung angeführtes Dokument
L : aus anderen Gründen angeführtes Dokument

.......................................................................................................
& : Mitglied der gleichen Patentfamilie, übereinstimmendes
      Dokument

EPO FORM 1503 03.82 (P04C03)

Europäisches
Patentamt

European
Patent Office

Office européen
des brevets

## EUROPÄISCHER RECHERCHENBERICHT

Nummer der Anmeldung

EP 15 15 9303

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | PAMELA FISCHER-POSOVSZKY ET AL: "A New Missense Mutation in the Leptin Gene Causes Mild Obesity and Hypogonadism without Affecting T Cell Responsiveness", JOURNAL OF CLINICAL ENDOCRINOLOGY & METABOLISM, Bd. 95, Nr. 6, 1. Juni 2010 (2010-06-01), Seiten 2836-2840, XP055196771, ISSN: 0021-972X, DOI: 10.1210/jc.2009-2466 * das ganze Dokument * | 1-16 | |
| A | MAZEN I ET AL: "A novel homozygous missense mutation of the leptin gene (N103K) in an obese Egyptian patient", MOLECULAR GENETICS AND METABOLISM, ACADEMIC PRESS, AMSTERDAM, NL, Bd. 97, Nr. 4, 1. August 2009 (2009-08-01) , Seiten 305-308, XP026282979, ISSN: 1096-7192, DOI: 10.1016/J.YMGME.2009.04.002 [gefunden am 2009-04-09] * das ganze Dokument * | 1-16 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 23. Juni 2015 | Weber, Peter |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **KELESIDIS T. et al.** Narrative Review: The Role of Leptin in Human Physiology: Emerging Clinical Applications. *Ann Intern Med.,* 19. Januar 2010, vol. 152 (2), 93-100
- **PAZ-FILHO G. et al.** Leptin treatment: Facts and expectations. *Metabolism,* 2014, 1-11
- **WABITSCH et al.** *N Engl J Med,* 01. Januar 2015, vol. 372 (1), 48-54
- **KRATZSCH et al.** A Rapid Quantitative Immunofunctional Assay for Measuring Human Leptin. *Horm Res,* 2002, vol. 57, 127-132
- **SAMBROOK et al.** Molecular cloning: a laboratory handbook. Corld Spring Harbor Laboratory Press, 1998

SEQUENCE LISTING

<110> Mediagnost Gesellschaft für Forschung und Herstellung von Diagnostika GmbH

<120> Zusammenstellung von Nachweisreagenzien, In-vitro-Verfahren zum Nachweisen von mutiertem Leptin und Verwendung eines Nachweisreagenzes

<130> E/53284EP/AW/kn

<160> 10

<170> BiSSAP 1.0

<210> 1
<211> 167
<212> PRT
<213> Homo sapiens

<220>
<221> SOURCE
<222> 1..167
<223> /mol_type="protein"
      /note="Leptin Precursor"
      /organism="Homo sapiens"

<400> 1
Met His Trp Gly Thr Leu Cys Gly Phe Leu Trp Leu Trp Pro Tyr Leu
1               5                   10                  15
Phe Tyr Val Gln Ala Val Pro Ile Gln Lys Val Gln Asp Asp Thr Lys
            20                  25                  30
Thr Leu Ile Lys Thr Ile Val Thr Arg Ile Asn Asp Ile Ser His Thr
        35                  40                  45
Gln Ser Val Ser Ser Lys Gln Lys Val Thr Gly Leu Asp Phe Ile Pro
    50                  55                  60
Gly Leu His Pro Ile Leu Thr Leu Ser Lys Met Asp Gln Thr Leu Ala
65                  70                  75                  80
Val Tyr Gln Gln Ile Leu Thr Ser Met Pro Ser Arg Asn Val Ile Gln
                85                  90                  95
Ile Ser Asn Asp Leu Glu Asn Leu Arg Asp Leu Leu His Val Leu Ala
            100                 105                 110
Phe Ser Lys Ser Cys His Leu Pro Trp Ala Ser Gly Leu Glu Thr Leu
        115                 120                 125
Asp Ser Leu Gly Gly Val Leu Glu Ala Ser Gly Tyr Ser Thr Glu Val
    130                 135                 140
Val Ala Leu Ser Arg Leu Gln Gly Ser Leu Gln Asp Met Leu Trp Gln
145                 150                 155                 160
Leu Asp Leu Ser Pro Gly Cys
                165

<210> 2
<211> 167
<212> PRT
<213> Homo sapiens

<220>
<221> SOURCE
<222> 1..167
<223> /mol_type="protein"
      /note="Leptin Precursor Variante D100Y"
      /organism="Homo sapiens"

<400> 2
Met His Trp Gly Thr Leu Cys Gly Phe Leu Trp Leu Trp Pro Tyr Leu
1               5                   10                  15
Phe Tyr Val Gln Ala Val Pro Ile Gln Lys Val Gln Asp Asp Thr Lys
            20                  25                  30
Thr Leu Ile Lys Thr Ile Val Thr Arg Ile Asn Asp Ile Ser His Thr
        35                  40                  45

```
Gln Ser Val Ser Ser Lys Gln Lys Val Thr Gly Leu Asp Phe Ile Pro
    50                  55                  60
Gly Leu His Pro Ile Leu Thr Leu Ser Lys Met Asp Gln Thr Leu Ala
65                  70                  75                  80
Val Tyr Gln Gln Ile Leu Thr Ser Met Pro Ser Arg Asn Val Ile Gln
                85                  90                  95
Ile Ser Asn Tyr Leu Glu Asn Leu Arg Asp Leu Leu His Val Leu Ala
            100                 105                 110
Phe Ser Lys Ser Cys His Leu Pro Trp Ala Ser Gly Leu Glu Thr Leu
            115                 120                 125
Asp Ser Leu Gly Gly Val Leu Glu Ala Ser Gly Tyr Ser Thr Glu Val
            130                 135                 140
Val Ala Leu Ser Arg Leu Gln Gly Ser Leu Gln Asp Met Leu Trp Gln
145                 150                 155                 160
Leu Asp Leu Ser Pro Gly Cys
                165


<210> 3
<211> 167
<212> PRT
<213> Homo sapiens


<220>
<221> SOURCE
<222> 1..167
<223> /mol_type="protein"
      /note="Leptin Precursor Variante N103K"
      /organism="Homo sapiens"


<400> 3
Met His Trp Gly Thr Leu Cys Gly Phe Leu Trp Leu Trp Pro Tyr Leu
1               5                   10                  15
Phe Tyr Val Gln Ala Val Pro Ile Gln Lys Val Gln Asp Asp Thr Lys
            20                  25                  30
Thr Leu Ile Lys Thr Ile Val Thr Arg Ile Asn Asp Ile Ser His Thr
            35                  40                  45
Gln Ser Val Ser Ser Lys Gln Lys Val Thr Gly Leu Asp Phe Ile Pro
    50                  55                  60
Gly Leu His Pro Ile Leu Thr Leu Ser Lys Met Asp Gln Thr Leu Ala
65                  70                  75                  80
Val Tyr Gln Gln Ile Leu Thr Ser Met Pro Ser Arg Asn Val Ile Gln
                85                  90                  95
Ile Ser Asn Asp Leu Glu Lys Leu Arg Asp Leu Leu His Val Leu Ala
            100                 105                 110
Phe Ser Lys Ser Cys His Leu Pro Trp Ala Ser Gly Leu Glu Thr Leu
            115                 120                 125
Asp Ser Leu Gly Gly Val Leu Glu Ala Ser Gly Tyr Ser Thr Glu Val
            130                 135                 140
Val Ala Leu Ser Arg Leu Gln Gly Ser Leu Gln Asp Met Leu Trp Gln
145                 150                 155                 160
Leu Asp Leu Ser Pro Gly Cys
                165


<210> 4
<211> 167
<212> PRT
<213> Homo sapiens


<220>
<221> SOURCE
<222> 1..167
<223> /mol_type="protein"
      /note="Leptin Precursor Variante L72S"
      /organism="Homo sapiens"


<400> 4
Met His Trp Gly Thr Leu Cys Gly Phe Leu Trp Leu Trp Pro Tyr Leu
1               5                   10                  15
```

Phe Tyr Val Gln Ala Val Pro Ile Gln Lys Val Gln Asp Asp Thr Lys
                20                  25                  30
Thr Leu Ile Lys Thr Ile Val Thr Arg Ile Asn Asp Ile Ser His Thr
            35                  40                  45
Gln Ser Val Ser Ser Lys Gln Lys Val Thr Gly Leu Asp Phe Ile Pro
        50                  55                  60
Gly Leu His Pro Ile Leu Thr Ser Ser Lys Met Asp Gln Thr Leu Ala
65                  70                  75                  80
Val Tyr Gln Gln Ile Leu Thr Ser Met Pro Ser Arg Asn Val Ile Gln
                85                  90                  95
Ile Ser Asn Asp Leu Glu Asn Leu Arg Asp Leu Leu His Val Leu Ala
                100                 105                 110
Phe Ser Lys Ser Cys His Leu Pro Trp Ala Ser Gly Leu Glu Thr Leu
            115                 120                 125
Asp Ser Leu Gly Gly Val Leu Glu Ala Ser Gly Tyr Ser Thr Glu Val
        130                 135                 140
Val Ala Leu Ser Arg Leu Gln Gly Ser Leu Gln Asp Met Leu Trp Gln
145                 150                 155                 160
Leu Asp Leu Ser Pro Gly Cys
                165

<210> 5
<211> 896
<212> PRT
<213> Homo sapiens

<220>
<221> SOURCE
<222> 1..896
<223> /mol_type="protein"
      /note="Leptinrezeptor Isoform A"
      /organism="Homo sapiens"

<400> 5
Met Ile Cys Gln Lys Phe Cys Val Val Leu Leu His Trp Glu Phe Ile
1               5                   10                  15
Tyr Val Ile Thr Ala Phe Asn Leu Ser Tyr Pro Ile Thr Pro Trp Arg
            20                  25                  30
Phe Lys Leu Ser Cys Met Pro Pro Asn Ser Thr Tyr Asp Tyr Phe Leu
            35                  40                  45
Leu Pro Ala Gly Leu Ser Lys Asn Thr Ser Asn Ser Asn Gly His Tyr
        50                  55                  60
Glu Thr Ala Val Glu Pro Lys Phe Asn Ser Ser Gly Thr His Phe Ser
65                  70                  75                  80
Asn Leu Ser Lys Thr Thr Phe His Cys Cys Phe Arg Ser Glu Gln Asp
                85                  90                  95
Arg Asn Cys Ser Leu Cys Ala Asp Asn Ile Glu Gly Lys Thr Phe Val
            100                 105                 110
Ser Thr Val Asn Ser Leu Val Phe Gln Gln Ile Asp Ala Asn Trp Asn
            115                 120                 125
Ile Gln Cys Trp Leu Lys Gly Asp Leu Lys Leu Phe Ile Cys Tyr Val
        130                 135                 140
Glu Ser Leu Phe Lys Asn Leu Phe Arg Asn Tyr Asn Tyr Lys Val His
145                 150                 155                 160
Leu Leu Tyr Val Leu Pro Glu Val Leu Glu Asp Ser Pro Leu Val Pro
                165                 170                 175
Gln Lys Gly Ser Phe Gln Met Val His Cys Asn Cys Ser Val His Glu
            180                 185                 190
Cys Cys Glu Cys Leu Val Pro Val Pro Thr Ala Lys Leu Asn Asp Thr
            195                 200                 205
Leu Leu Met Cys Leu Lys Ile Thr Ser Gly Gly Val Ile Phe Gln Ser
        210                 215                 220
Pro Leu Met Ser Val Gln Pro Ile Asn Met Val Lys Pro Asp Pro Pro
225                 230                 235                 240
Leu Gly Leu His Met Glu Ile Thr Asp Asp Gly Asn Leu Lys Ile Ser
                245                 250                 255
Trp Ser Ser Pro Pro Leu Val Pro Phe Pro Leu Gln Tyr Gln Val Lys
            260                 265                 270

```
Tyr Ser Glu Asn Ser Thr Thr Val Ile Arg Glu Ala Asp Lys Ile Val
        275                 280                 285
Ser Ala Thr Ser Leu Leu Val Asp Ser Ile Leu Pro Gly Ser Ser Tyr
    290                 295                 300
Glu Val Gln Val Arg Gly Lys Arg Leu Asp Gly Pro Gly Ile Trp Ser
305                 310                 315                 320
Asp Trp Ser Thr Pro Arg Val Phe Thr Thr Gln Asp Val Ile Tyr Phe
            325                 330                 335
Pro Pro Lys Ile Leu Thr Ser Val Gly Ser Asn Val Ser Phe His Cys
            340                 345                 350
Ile Tyr Lys Lys Glu Asn Lys Ile Val Pro Ser Lys Glu Ile Val Trp
        355                 360                 365
Trp Met Asn Leu Ala Glu Lys Ile Pro Gln Ser Gln Tyr Asp Val Val
370                 375                 380
Ser Asp His Val Ser Lys Val Thr Phe Phe Asn Leu Asn Glu Thr Lys
385                 390                 395                 400
Pro Arg Gly Lys Phe Thr Tyr Asp Ala Val Tyr Cys Cys Asn Glu His
            405                 410                 415
Glu Cys His His Arg Tyr Ala Glu Leu Tyr Val Ile Asp Val Asn Ile
            420                 425                 430
Asn Ile Ser Cys Glu Thr Asp Gly Tyr Leu Thr Lys Met Thr Cys Arg
        435                 440                 445
Trp Ser Thr Ser Thr Ile Gln Ser Leu Ala Glu Ser Thr Leu Gln Leu
    450                 455                 460
Arg Tyr His Arg Ser Ser Leu Tyr Cys Ser Asp Ile Pro Ser Ile His
465                 470                 475                 480
Pro Ile Ser Glu Pro Lys Asp Cys Tyr Leu Gln Ser Asp Gly Phe Tyr
            485                 490                 495
Glu Cys Ile Phe Gln Pro Ile Phe Leu Leu Ser Gly Tyr Thr Met Trp
            500                 505                 510
Ile Arg Ile Asn His Ser Leu Gly Ser Leu Asp Ser Pro Pro Thr Cys
    515                 520                 525
Val Leu Pro Asp Ser Val Val Lys Pro Leu Pro Pro Ser Ser Val Lys
    530                 535                 540
Ala Glu Ile Thr Ile Asn Ile Gly Leu Leu Lys Ile Ser Trp Glu Lys
545                 550                 555                 560
Pro Val Phe Pro Glu Asn Asn Leu Gln Phe Gln Ile Arg Tyr Gly Leu
            565                 570                 575
Ser Gly Lys Glu Val Gln Trp Lys Met Tyr Glu Val Tyr Asp Ala Lys
        580                 585                 590
Ser Lys Ser Val Ser Leu Pro Val Pro Asp Leu Cys Ala Val Tyr Ala
    595                 600                 605
Val Gln Val Arg Cys Lys Arg Leu Asp Gly Leu Gly Tyr Trp Ser Asn
    610                 615                 620
Trp Ser Asn Pro Ala Tyr Thr Val Val Met Asp Ile Lys Val Pro Met
625                 630                 635                 640
Arg Gly Pro Glu Phe Trp Arg Ile Ile Asn Gly Asp Thr Met Lys Lys
            645                 650                 655
Glu Lys Asn Val Thr Leu Leu Trp Lys Pro Leu Met Lys Asn Asp Ser
            660                 665                 670
Leu Cys Ser Val Gln Arg Tyr Val Ile Asn His His Thr Ser Cys Asn
        675                 680                 685
Gly Thr Trp Ser Glu Asp Val Gly Asn His Thr Lys Phe Thr Phe Leu
    690                 695                 700
Trp Thr Glu Gln Ala His Thr Val Thr Val Leu Ala Ile Asn Ser Ile
705                 710                 715                 720
Gly Ala Ser Val Ala Asn Phe Asn Leu Thr Phe Ser Trp Pro Met Ser
            725                 730                 735
Lys Val Asn Ile Val Gln Ser Leu Ser Ala Tyr Pro Leu Asn Ser Ser
            740                 745                 750
Cys Val Ile Val Ser Trp Ile Leu Ser Pro Ser Asp Tyr Lys Leu Met
        755                 760                 765
Tyr Phe Ile Ile Glu Trp Lys Asn Leu Asn Glu Asp Gly Glu Ile Lys
    770                 775                 780
Trp Leu Arg Ile Ser Ser Ser Val Lys Lys Tyr Tyr Ile His Asp His
785                 790                 795                 800
Phe Ile Pro Ile Glu Lys Tyr Gln Phe Ser Leu Tyr Pro Ile Phe Met
            805                 810                 815
```

Glu Gly Val Gly Lys Pro Lys Ile Ile Asn Ser Phe Thr Gln Asp Asp
820 825 830
Ile Glu Lys His Gln Ser Asp Ala Gly Leu Tyr Val Ile Val Pro Val
835 840 845
Ile Ile Ser Ser Ser Ile Leu Leu Leu Gly Thr Leu Leu Ile Ser His
850 855 860
Gln Arg Met Lys Lys Leu Phe Trp Glu Asp Val Pro Asn Pro Lys Asn
865 870 875 880
Cys Ser Trp Ala Gln Gly Leu Asn Phe Gln Lys Arg Thr Asp Ile Leu
885 890 895

<210> 6
<211> 1165
<212> PRT
<213> Homo sapiens

<220>
<221> SOURCE
<222> 1..1165
<223> /mol_type="protein"
      /note="Leptinrezeptor Isoform B"
      /organism="Homo sapiens"

<400> 6
Met Ile Cys Gln Lys Phe Cys Val Val Leu Leu His Trp Glu Phe Ile
1 5 10 15
Tyr Val Ile Thr Ala Phe Asn Leu Ser Tyr Pro Ile Thr Pro Trp Arg
20 25 30
Phe Lys Leu Ser Cys Met Pro Pro Asn Ser Thr Tyr Asp Tyr Phe Leu
35 40 45
Leu Pro Ala Gly Leu Ser Lys Asn Thr Ser Asn Ser Asn Gly His Tyr
50 55 60
Glu Thr Ala Val Glu Pro Lys Phe Asn Ser Ser Gly Thr His Phe Ser
65 70 75 80
Asn Leu Ser Lys Thr Thr Phe His Cys Cys Phe Arg Ser Glu Gln Asp
85 90 95
Arg Asn Cys Ser Leu Cys Ala Asp Asn Ile Glu Gly Lys Thr Phe Val
100 105 110
Ser Thr Val Asn Ser Leu Val Phe Gln Gln Ile Asp Ala Asn Trp Asn
115 120 125
Ile Gln Cys Trp Leu Lys Gly Asp Leu Lys Leu Phe Ile Cys Tyr Val
130 135 140
Glu Ser Leu Phe Lys Asn Leu Phe Arg Asn Tyr Asn Tyr Lys Val His
145 150 155 160
Leu Leu Tyr Val Leu Pro Glu Val Leu Glu Asp Ser Pro Leu Val Pro
165 170 175
Gln Lys Gly Ser Phe Gln Met Val His Cys Asn Cys Ser Val His Glu
180 185 190
Cys Cys Glu Cys Leu Val Pro Val Pro Thr Ala Lys Leu Asn Asp Thr
195 200 205
Leu Leu Met Cys Leu Lys Ile Thr Ser Gly Gly Val Ile Phe Gln Ser
210 215 220
Pro Leu Met Ser Val Gln Pro Ile Asn Met Val Lys Pro Asp Pro Pro
225 230 235 240
Leu Gly Leu His Met Glu Ile Thr Asp Asp Gly Asn Leu Lys Ile Ser
245 250 255
Trp Ser Ser Pro Pro Leu Val Pro Phe Pro Leu Gln Tyr Gln Val Lys
260 265 270
Tyr Ser Glu Asn Ser Thr Thr Val Ile Arg Glu Ala Asp Lys Ile Val
275 280 285
Ser Ala Thr Ser Leu Leu Val Asp Ser Ile Leu Pro Gly Ser Ser Tyr
290 295 300
Glu Val Gln Val Arg Gly Lys Arg Leu Asp Gly Pro Gly Ile Trp Ser
305 310 315 320
Asp Trp Ser Thr Pro Arg Val Phe Thr Thr Gln Asp Val Ile Tyr Phe
325 330 335
Pro Pro Lys Ile Leu Thr Ser Val Gly Ser Asn Val Ser Phe His Cys
340 345 350

```
Ile Tyr Lys Lys Glu Asn Lys Ile Val Pro Ser Lys Glu Ile Val Trp
        355                 360                 365
Trp Met Asn Leu Ala Glu Lys Ile Pro Gln Ser Gln Tyr Asp Val Val
    370                 375                 380
Ser Asp His Val Ser Lys Val Thr Phe Phe Asn Leu Asn Glu Thr Lys
385                 390                 395                 400
Pro Arg Gly Lys Phe Thr Tyr Asp Ala Val Tyr Cys Cys Asn Glu His
                405                 410                 415
Glu Cys His His Arg Tyr Ala Glu Leu Tyr Val Ile Asp Val Asn Ile
            420                 425                 430
Asn Ile Ser Cys Glu Thr Asp Gly Tyr Leu Thr Lys Met Thr Cys Arg
        435                 440                 445
Trp Ser Thr Ser Thr Ile Gln Ser Leu Ala Glu Ser Thr Leu Gln Leu
    450                 455                 460
Arg Tyr His Arg Ser Ser Leu Tyr Cys Ser Asp Ile Pro Ser Ile His
465                 470                 475                 480
Pro Ile Ser Glu Pro Lys Asp Cys Tyr Leu Gln Ser Asp Gly Phe Tyr
                485                 490                 495
Glu Cys Ile Phe Gln Pro Ile Phe Leu Leu Ser Gly Tyr Thr Met Trp
            500                 505                 510
Ile Arg Ile Asn His Ser Leu Gly Ser Leu Asp Ser Pro Pro Thr Cys
        515                 520                 525
Val Leu Pro Asp Ser Val Val Lys Pro Leu Pro Pro Ser Ser Val Lys
    530                 535                 540
Ala Glu Ile Thr Ile Asn Ile Gly Leu Leu Lys Ile Ser Trp Glu Lys
545                 550                 555                 560
Pro Val Phe Pro Glu Asn Asn Leu Gln Phe Gln Ile Arg Tyr Gly Leu
                565                 570                 575
Ser Gly Lys Glu Val Gln Trp Lys Met Tyr Glu Val Tyr Asp Ala Lys
            580                 585                 590
Ser Lys Ser Val Ser Leu Pro Val Pro Asp Leu Cys Ala Val Tyr Ala
        595                 600                 605
Val Gln Val Arg Cys Lys Arg Leu Asp Gly Leu Gly Tyr Trp Ser Asn
    610                 615                 620
Trp Ser Asn Pro Ala Tyr Thr Val Val Met Asp Ile Lys Val Pro Met
625                 630                 635                 640
Arg Gly Pro Glu Phe Trp Arg Ile Ile Asn Gly Asp Thr Met Lys Lys
                645                 650                 655
Glu Lys Asn Val Thr Leu Leu Trp Lys Pro Leu Met Lys Asn Asp Ser
            660                 665                 670
Leu Cys Ser Val Gln Arg Tyr Val Ile Asn His His Thr Ser Cys Asn
        675                 680                 685
Gly Thr Trp Ser Glu Asp Val Gly Asn His Thr Lys Phe Thr Phe Leu
        690                 695                 700
Trp Thr Glu Gln Ala His Thr Val Thr Val Leu Ala Ile Asn Ser Ile
705                 710                 715                 720
Gly Ala Ser Val Ala Asn Phe Asn Leu Thr Phe Ser Trp Pro Met Ser
                725                 730                 735
Lys Val Asn Ile Val Gln Ser Leu Ser Ala Tyr Pro Leu Asn Ser Ser
            740                 745                 750
Cys Val Ile Val Ser Trp Ile Leu Ser Pro Ser Asp Tyr Lys Leu Met
        755                 760                 765
Tyr Phe Ile Ile Glu Trp Lys Asn Leu Asn Glu Asp Gly Glu Ile Lys
    770                 775                 780
Trp Leu Arg Ile Ser Ser Ser Val Lys Lys Tyr Tyr Ile His Asp His
785                 790                 795                 800
Phe Ile Pro Ile Glu Lys Tyr Gln Phe Ser Leu Tyr Pro Ile Phe Met
                805                 810                 815
Glu Gly Val Gly Lys Pro Lys Ile Ile Asn Ser Phe Thr Gln Asp Asp
            820                 825                 830
Ile Glu Lys His Gln Ser Asp Ala Gly Leu Tyr Val Ile Val Pro Val
        835                 840                 845
Ile Ile Ser Ser Ser Ile Leu Leu Leu Gly Thr Leu Leu Ile Ser His
        850                 855                 860
Gln Arg Met Lys Lys Leu Phe Trp Glu Asp Val Pro Asn Pro Lys Asn
865                 870                 875                 880
Cys Ser Trp Ala Gln Gly Leu Asn Phe Gln Lys Pro Glu Thr Phe Glu
                885                 890                 895
```

17

```
His Leu Phe Ile Lys His Thr Ala Ser Val Thr Cys Gly Pro Leu Leu
            900                     905                 910
Leu Glu Pro Glu Thr Ile Ser Glu Asp Ile Ser Val Asp Thr Ser Trp
            915                 920                 925
Lys Asn Lys Asp Glu Met Met Pro Thr Thr Val Val Ser Leu Leu Ser
    930                 935                 940
Thr Thr Asp Leu Glu Lys Gly Ser Val Cys Ile Ser Asp Gln Phe Asn
945                 950                 955                 960
Ser Val Asn Phe Ser Glu Ala Glu Gly Thr Glu Val Thr Tyr Glu Asp
                965                 970                 975
Glu Ser Gln Arg Gln Pro Phe Val Lys Tyr Ala Thr Leu Ile Ser Asn
            980                 985                 990
Ser Lys Pro Ser Glu Thr Gly Glu Glu Gln Gly Leu Ile Asn Ser Ser
            995                 1000                1005
Val Thr Lys Cys Phe Ser Ser Lys Asn Ser Pro Leu Lys Asp Ser Phe
    1010                1015                1020
Ser Asn Ser Ser Trp Glu Ile Glu Ala Gln Ala Phe Phe Ile Leu Ser
1025                1030                1035                1040
Asp Gln His Pro Asn Ile Ile Ser Pro His Leu Thr Phe Ser Glu Gly
                1045                1050                1055
Leu Asp Glu Leu Leu Lys Leu Glu Gly Asn Phe Pro Glu Glu Asn Asn
                1060                1065                1070
Asp Lys Lys Ser Ile Tyr Tyr Leu Gly Val Thr Ser Ile Lys Lys Arg
            1075                1080                1085
Glu Ser Gly Val Leu Leu Thr Asp Lys Ser Arg Val Ser Cys Pro Phe
        1090                1095                1100
Pro Ala Pro Cys Leu Phe Thr Asp Ile Arg Val Leu Gln Asp Ser Cys
1105                1110                1115                1120
Ser His Phe Val Glu Asn Asn Ile Asn Leu Gly Thr Ser Ser Lys Lys
                1125                1130                1135
Thr Phe Ala Ser Tyr Met Pro Gln Phe Gln Thr Cys Ser Thr Gln Thr
                1140                1145                1150
His Lys Ile Met Glu Asn Lys Met Cys Asp Leu Thr Val
            1155                1160                1165
```

```
<210> 7
<211> 958
<212> PRT
<213> Homo sapiens

<220>
<221> SOURCE
<222> 1..958
<223> /mol_type="protein"
      /note="Leptinrezeptor Isoform C"
      /organism="Homo sapiens"

<400> 7
Met Ile Cys Gln Lys Phe Cys Val Val Leu Leu His Trp Glu Phe Ile
1               5               10                  15
Tyr Val Ile Thr Ala Phe Asn Leu Ser Tyr Pro Ile Thr Pro Trp Arg
            20                  25                  30
Phe Lys Leu Ser Cys Met Pro Pro Asn Ser Thr Tyr Asp Tyr Phe Leu
        35                  40                  45
Leu Pro Ala Gly Leu Ser Lys Asn Thr Ser Asn Ser Asn Gly His Tyr
    50                  55                  60
Glu Thr Ala Val Glu Pro Lys Phe Asn Ser Ser Gly Thr His Phe Ser
65                  70                  75                  80
Asn Leu Ser Lys Thr Thr Phe His Cys Cys Phe Arg Ser Glu Gln Asp
                85                  90                  95
Arg Asn Cys Ser Leu Cys Ala Asp Asn Ile Glu Gly Lys Thr Phe Val
            100                 105                 110
Ser Thr Val Asn Ser Leu Val Phe Gln Gln Ile Asp Ala Asn Trp Asn
        115                 120                 125
Ile Gln Cys Trp Leu Lys Gly Asp Leu Lys Leu Phe Ile Cys Tyr Val
    130                 135                 140
Glu Ser Leu Phe Lys Asn Leu Phe Arg Asn Tyr Asn Tyr Lys Val His
145                 150                 155                 160
```

Leu Leu Tyr Val Leu Pro Glu Val Leu Glu Asp Ser Pro Leu Val Pro
165 170 175
Gln Lys Gly Ser Phe Gln Met Val His Cys Asn Cys Ser Val His Glu
180 185 190
Cys Cys Glu Cys Leu Val Pro Val Pro Thr Ala Lys Leu Asn Asp Thr
195 200 205
Leu Leu Met Cys Leu Lys Ile Thr Ser Gly Gly Val Ile Phe Gln Ser
210 215 220
Pro Leu Met Ser Val Gln Pro Ile Asn Met Val Lys Pro Asp Pro Pro
225 230 235 240
Leu Gly Leu His Met Glu Ile Thr Asp Asp Gly Asn Leu Lys Ile Ser
245 250 255
Trp Ser Ser Pro Pro Leu Val Pro Phe Pro Leu Gln Tyr Gln Val Lys
260 265 270
Tyr Ser Glu Asn Ser Thr Thr Val Ile Arg Glu Ala Asp Lys Ile Val
275 280 285
Ser Ala Thr Ser Leu Leu Val Asp Ser Ile Leu Pro Gly Ser Ser Tyr
290 295 300
Glu Val Gln Val Arg Gly Lys Arg Leu Asp Gly Pro Gly Ile Trp Ser
305 310 315 320
Asp Trp Ser Thr Pro Arg Val Phe Thr Thr Gln Asp Val Ile Tyr Phe
325 330 335
Pro Pro Lys Ile Leu Thr Ser Val Gly Ser Asn Val Ser Phe His Cys
340 345 350
Ile Tyr Lys Lys Glu Asn Lys Ile Val Pro Ser Lys Glu Ile Val Trp
355 360 365
Trp Met Asn Leu Ala Glu Lys Ile Pro Gln Ser Gln Tyr Asp Val Val
370 375 380
Ser Asp His Val Ser Lys Val Thr Phe Phe Asn Leu Asn Glu Thr Lys
385 390 395 400
Pro Arg Gly Lys Phe Thr Tyr Asp Ala Val Tyr Cys Cys Asn Glu His
405 410 415
Glu Cys His His Arg Tyr Ala Glu Leu Tyr Val Ile Asp Val Asn Ile
420 425 430
Asn Ile Ser Cys Glu Thr Asp Gly Tyr Leu Thr Lys Met Thr Cys Arg
435 440 445
Trp Ser Thr Ser Thr Ile Gln Ser Leu Ala Glu Ser Thr Leu Gln Leu
450 455 460
Arg Tyr His Arg Ser Ser Leu Tyr Cys Ser Asp Ile Pro Ser Ile His
465 470 475 480
Pro Ile Ser Glu Pro Lys Asp Cys Tyr Leu Gln Ser Asp Gly Phe Tyr
485 490 495
Glu Cys Ile Phe Gln Pro Ile Phe Leu Leu Ser Gly Tyr Thr Met Trp
500 505 510
Ile Arg Ile Asn His Ser Leu Gly Ser Leu Asp Ser Pro Pro Thr Cys
515 520 525
Val Leu Pro Asp Ser Val Val Lys Pro Leu Pro Pro Ser Ser Val Lys
530 535 540
Ala Glu Ile Thr Ile Asn Ile Gly Leu Leu Lys Ile Ser Trp Glu Lys
545 550 555 560
Pro Val Phe Pro Glu Asn Asn Leu Gln Phe Gln Ile Arg Tyr Gly Leu
565 570 575
Ser Gly Lys Glu Val Gln Trp Lys Met Tyr Glu Val Tyr Asp Ala Lys
580 585 590
Ser Lys Ser Val Ser Leu Pro Val Pro Asp Leu Cys Ala Val Tyr Ala
595 600 605
Val Gln Val Arg Cys Lys Arg Leu Asp Gly Leu Gly Tyr Trp Ser Asn
610 615 620
Trp Ser Asn Pro Ala Tyr Thr Val Val Met Asp Ile Lys Val Pro Met
625 630 635 640
Arg Gly Pro Glu Phe Trp Arg Ile Ile Asn Gly Asp Thr Met Lys Lys
645 650 655
Glu Lys Asn Val Thr Leu Leu Trp Lys Pro Leu Met Lys Asn Asp Ser
660 665 670
Leu Cys Ser Val Gln Arg Tyr Val Ile Asn His His Thr Ser Cys Asn
675 680 685
Gly Thr Trp Ser Glu Asp Val Gly Asn His Thr Lys Phe Thr Phe Leu
690 695 700

```
Trp Thr Glu Gln Ala His Thr Val Thr Val Leu Ala Ile Asn Ser Ile
705                 710                 715                 720
Gly Ala Ser Val Ala Asn Phe Asn Leu Thr Phe Ser Trp Pro Met Ser
            725                 730                 735
Lys Val Asn Ile Val Gln Ser Leu Ser Ala Tyr Pro Leu Asn Ser Ser
            740                 745                 750
Cys Val Ile Val Ser Trp Ile Leu Ser Pro Ser Asp Tyr Lys Leu Met
        755                 760                 765
Tyr Phe Ile Ile Glu Trp Lys Asn Leu Asn Glu Asp Gly Glu Ile Lys
        770                 775                 780
Trp Leu Arg Ile Ser Ser Ser Val Lys Lys Tyr Tyr Ile His Asp His
785                 790                 795                 800
Phe Ile Pro Ile Glu Lys Tyr Gln Phe Ser Leu Tyr Pro Ile Phe Met
                805                 810                 815
Glu Gly Val Gly Lys Pro Lys Ile Ile Asn Ser Phe Thr Gln Asp Asp
            820                 825                 830
Ile Glu Lys His Gln Ser Asp Ala Gly Leu Tyr Val Ile Val Pro Val
        835                 840                 845
Ile Ile Ser Ser Ser Ile Leu Leu Leu Gly Thr Leu Leu Ile Ser His
        850                 855                 860
Gln Arg Met Lys Lys Leu Phe Trp Glu Asp Val Pro Asn Pro Lys Asn
865                 870                 875                 880
Cys Ser Trp Ala Gln Gly Leu Asn Phe Gln Lys Met Leu Glu Gly Ser
                885                 890                 895
Met Phe Val Lys Ser His His His Ser Leu Ile Ser Ser Thr Gln Gly
            900                 905                 910
His Lys His Cys Gly Arg Pro Gln Gly Pro Leu His Arg Lys Thr Arg
        915                 920                 925
Asp Leu Cys Ser Leu Val Tyr Leu Leu Thr Leu Pro Pro Leu Leu Ser
        930                 935                 940
Tyr Asp Pro Ala Lys Ser Pro Ser Val Arg Asn Thr Gln Glu
945                 950                 955

<210> 8
<211> 906
<212> PRT
<213> Homo sapiens

<220>
<221> SOURCE
<222> 1..906
<223> /mol_type="protein"
      /note="Leptinrezeptor Isoform D"
      /organism="Homo sapiens"

<400> 8
Met Ile Cys Gln Lys Phe Cys Val Val Leu Leu His Trp Glu Phe Ile
1                 5                   10                  15
Tyr Val Ile Thr Ala Phe Asn Leu Ser Tyr Pro Ile Thr Pro Trp Arg
            20                  25                  30
Phe Lys Leu Ser Cys Met Pro Pro Asn Ser Thr Tyr Asp Tyr Phe Leu
        35                  40                  45
Leu Pro Ala Gly Leu Ser Lys Asn Thr Ser Asn Ser Asn Gly His Tyr
        50                  55                  60
Glu Thr Ala Val Glu Pro Lys Phe Asn Ser Ser Gly Thr His Phe Ser
65                  70                  75                  80
Asn Leu Ser Lys Thr Thr Phe His Cys Cys Phe Arg Ser Glu Gln Asp
                85                  90                  95
Arg Asn Cys Ser Leu Cys Ala Asp Asn Ile Glu Gly Lys Thr Phe Val
            100                 105                 110
Ser Thr Val Asn Ser Leu Val Phe Gln Gln Ile Asp Ala Asn Trp Asn
        115                 120                 125
Ile Gln Cys Trp Leu Lys Gly Asp Leu Lys Leu Phe Ile Cys Tyr Val
        130                 135                 140
Glu Ser Leu Phe Lys Asn Leu Phe Arg Asn Tyr Asn Tyr Lys Val His
145                 150                 155                 160
Leu Leu Tyr Val Leu Pro Glu Val Leu Glu Asp Ser Pro Leu Val Pro
                165                 170                 175
```

```
Gln Lys Gly Ser Phe Gln Met Val His Cys Asn Cys Ser Val His Glu
        180                 185                 190
Cys Cys Glu Cys Leu Val Pro Val Pro Thr Ala Lys Leu Asn Asp Thr
        195                 200                 205
Leu Leu Met Cys Leu Lys Ile Thr Ser Gly Gly Val Ile Phe Gln Ser
    210                 215                 220
Pro Leu Met Ser Val Gln Pro Ile Asn Met Val Lys Pro Asp Pro Pro
225                 230                 235                 240
Leu Gly Leu His Met Glu Ile Thr Asp Asp Gly Asn Leu Lys Ile Ser
                245                 250                 255
Trp Ser Ser Pro Pro Leu Val Pro Phe Pro Leu Gln Tyr Gln Val Lys
            260                 265                 270
Tyr Ser Glu Asn Ser Thr Thr Val Ile Arg Glu Ala Asp Lys Ile Val
        275                 280                 285
Ser Ala Thr Ser Leu Leu Val Asp Ser Ile Leu Pro Gly Ser Ser Tyr
    290                 295                 300
Glu Val Gln Val Arg Gly Lys Arg Leu Asp Gly Pro Gly Ile Trp Ser
305                 310                 315                 320
Asp Trp Ser Thr Pro Arg Val Phe Thr Thr Gln Asp Val Ile Tyr Phe
                325                 330                 335
Pro Pro Lys Ile Leu Thr Ser Val Gly Ser Asn Val Ser Phe His Cys
            340                 345                 350
Ile Tyr Lys Lys Glu Asn Lys Ile Val Pro Ser Lys Glu Ile Val Trp
        355                 360                 365
Trp Met Asn Leu Ala Glu Lys Ile Pro Gln Ser Gln Tyr Asp Val Val
    370                 375                 380
Ser Asp His Val Ser Lys Val Thr Phe Phe Asn Leu Asn Glu Thr Lys
385                 390                 395                 400
Pro Arg Gly Lys Phe Thr Tyr Asp Ala Val Tyr Cys Cys Asn Glu His
                405                 410                 415
Glu Cys His His Arg Tyr Ala Glu Leu Tyr Val Ile Asp Val Asn Ile
            420                 425                 430
Asn Ile Ser Cys Glu Thr Asp Gly Tyr Leu Thr Lys Met Thr Cys Arg
        435                 440                 445
Trp Ser Thr Ser Thr Ile Gln Ser Leu Ala Glu Ser Thr Leu Gln Leu
    450                 455                 460
Arg Tyr His Arg Ser Ser Leu Tyr Cys Ser Asp Ile Pro Ser Ile His
465                 470                 475                 480
Pro Ile Ser Glu Pro Lys Asp Cys Tyr Leu Gln Ser Asp Gly Phe Tyr
                485                 490                 495
Glu Cys Ile Phe Gln Pro Ile Phe Leu Leu Ser Gly Tyr Thr Met Trp
            500                 505                 510
Ile Arg Ile Asn His Ser Leu Gly Ser Leu Asp Ser Pro Pro Thr Cys
        515                 520                 525
Val Leu Pro Asp Ser Val Val Lys Pro Leu Pro Pro Ser Ser Val Lys
    530                 535                 540
Ala Glu Ile Thr Ile Asn Ile Gly Leu Leu Lys Ile Ser Trp Glu Lys
545                 550                 555                 560
Pro Val Phe Pro Glu Asn Asn Leu Gln Phe Gln Ile Arg Tyr Gly Leu
                565                 570                 575
Ser Gly Lys Glu Val Gln Trp Lys Met Tyr Glu Val Tyr Asp Ala Lys
            580                 585                 590
Ser Lys Ser Val Ser Leu Pro Val Pro Asp Leu Cys Ala Val Tyr Ala
        595                 600                 605
Val Gln Val Arg Cys Lys Arg Leu Asp Gly Leu Gly Tyr Trp Ser Asn
    610                 615                 620
Trp Ser Asn Pro Ala Tyr Thr Val Val Met Asp Ile Lys Val Pro Met
625                 630                 635                 640
Arg Gly Pro Glu Phe Trp Arg Ile Ile Asn Gly Asp Thr Met Lys Lys
                645                 650                 655
Glu Lys Asn Val Thr Leu Leu Trp Lys Pro Leu Met Lys Asn Asp Ser
            660                 665                 670
Leu Cys Ser Val Gln Arg Tyr Val Ile Asn His His Thr Ser Cys Asn
        675                 680                 685
Gly Thr Trp Ser Glu Asp Val Gly Asn His Thr Lys Phe Thr Phe Leu
    690                 695                 700
Trp Thr Glu Gln Ala His Thr Val Thr Val Leu Ala Ile Asn Ser Ile
705                 710                 715                 720
```

Gly Ala Ser Val Ala Asn Phe Asn Leu Thr Phe Ser Trp Pro Met Ser
                725                 730                 735
Lys Val Asn Ile Val Gln Ser Leu Ser Ala Tyr Pro Leu Asn Ser Ser
            740                 745                 750
Cys Val Ile Val Ser Trp Ile Leu Ser Pro Ser Asp Tyr Lys Leu Met
            755                 760                 765
Tyr Phe Ile Ile Glu Trp Lys Asn Leu Asn Glu Asp Gly Glu Ile Lys
        770                 775                 780
Trp Leu Arg Ile Ser Ser Ser Val Lys Lys Tyr Tyr Ile His Asp His
785                 790                 795                 800
Phe Ile Pro Ile Glu Lys Tyr Gln Phe Ser Leu Tyr Pro Ile Phe Met
                805                 810                 815
Glu Gly Val Gly Lys Pro Lys Ile Ile Asn Ser Phe Thr Gln Asp Asp
            820                 825                 830
Ile Glu Lys His Gln Ser Asp Ala Gly Leu Tyr Val Ile Val Pro Val
            835                 840                 845
Ile Ile Ser Ser Ser Ile Leu Leu Leu Gly Thr Leu Leu Ile Ser His
        850                 855                 860
Gln Arg Met Lys Lys Leu Phe Trp Glu Asp Val Pro Asn Pro Lys Asn
865                 870                 875                 880
Cys Ser Trp Ala Gln Gly Leu Asn Phe Gln Lys Lys Met Pro Gly Thr
                885                 890                 895
Lys Glu Leu Leu Gly Gly Gly Trp Leu Thr
                900                 905

<210> 9
<211> 839
<212> PRT
<213> Homo sapiens

<220>
<221> SOURCE
<222> 1..839
<223> /mol_type="protein"
      /note="Leptinrezeptor Isoform E"
      /organism="Homo sapiens"

<400> 9
Met Ile Cys Gln Lys Phe Cys Val Val Leu Leu His Trp Glu Phe Ile
1               5                   10                  15
Tyr Val Ile Thr Ala Phe Asn Leu Ser Tyr Pro Ile Thr Pro Trp Arg
            20                  25                  30
Phe Lys Leu Ser Cys Met Pro Pro Asn Ser Thr Tyr Asp Tyr Phe Leu
        35                  40                  45
Leu Pro Ala Gly Leu Ser Lys Asn Thr Ser Asn Ser Asn Gly His Tyr
        50                  55                  60
Glu Thr Ala Val Glu Pro Lys Phe Asn Ser Ser Gly Thr His Phe Ser
65                  70                  75                  80
Asn Leu Ser Lys Thr Thr Phe His Cys Cys Phe Arg Ser Glu Gln Asp
                85                  90                  95
Arg Asn Cys Ser Leu Cys Ala Asp Asn Ile Glu Gly Lys Thr Phe Val
            100                 105                 110
Ser Thr Val Asn Ser Leu Val Phe Gln Gln Ile Asp Ala Asn Trp Asn
            115                 120                 125
Ile Gln Cys Trp Leu Lys Gly Asp Leu Lys Leu Phe Ile Cys Tyr Val
        130                 135                 140
Glu Ser Leu Phe Lys Asn Leu Phe Arg Asn Tyr Asn Tyr Lys Val His
145                 150                 155                 160
Leu Leu Tyr Val Leu Pro Glu Val Leu Glu Asp Ser Pro Leu Val Pro
                165                 170                 175
Gln Lys Gly Ser Phe Gln Met Val His Cys Asn Cys Ser Val His Glu
            180                 185                 190
Cys Cys Glu Cys Leu Val Pro Val Pro Thr Ala Lys Leu Asn Asp Thr
        195                 200                 205
Leu Leu Met Cys Leu Lys Ile Thr Ser Gly Gly Val Ile Phe Gln Ser
        210                 215                 220
Pro Leu Met Ser Val Gln Pro Ile Asn Met Val Lys Pro Asp Pro Pro
225                 230                 235                 240

```
Leu Gly Leu His Met Glu Ile Thr Asp Asp Gly Asn Leu Lys Ile Ser
            245               250                   255
Trp Ser Ser Pro Pro Leu Val Pro Phe Pro Leu Gln Tyr Gln Val Lys
            260               265                   270
Tyr Ser Glu Asn Ser Thr Thr Val Ile Arg Glu Ala Asp Lys Ile Val
            275               280                   285
Ser Ala Thr Ser Leu Leu Val Asp Ser Ile Leu Pro Gly Ser Ser Tyr
            290               295                   300
Glu Val Gln Val Arg Gly Lys Arg Leu Asp Gly Pro Gly Ile Trp Ser
305               310               315                   320
Asp Trp Ser Thr Pro Arg Val Phe Thr Thr Gln Asp Val Ile Tyr Phe
            325               330                   335
Pro Pro Lys Ile Leu Thr Ser Val Gly Ser Asn Val Ser Phe His Cys
            340               345                   350
Ile Tyr Lys Lys Glu Asn Lys Ile Val Pro Ser Lys Glu Ile Val Trp
            355               360                   365
Trp Met Asn Leu Ala Glu Lys Ile Pro Gln Ser Gln Tyr Asp Val Val
            370               375                   380
Ser Asp His Val Ser Lys Val Thr Phe Phe Asn Leu Asn Glu Thr Lys
385               390               395                   400
Pro Arg Gly Lys Phe Thr Tyr Asp Ala Val Tyr Cys Cys Asn Glu His
            405               410                   415
Glu Cys His His Arg Tyr Ala Glu Leu Tyr Val Ile Asp Val Asn Ile
            420               425                   430
Asn Ile Ser Cys Glu Thr Asp Gly Tyr Leu Thr Lys Met Thr Cys Arg
            435               440                   445
Trp Ser Thr Ser Thr Ile Gln Ser Leu Ala Glu Ser Thr Leu Gln Leu
            450               455               460
Arg Tyr His Arg Ser Ser Leu Tyr Cys Ser Asp Ile Pro Ser Ile His
465               470               475                   480
Pro Ile Ser Glu Pro Lys Asp Cys Tyr Leu Gln Ser Asp Gly Phe Tyr
            485               490                   495
Glu Cys Ile Phe Gln Pro Ile Phe Leu Leu Ser Gly Tyr Thr Met Trp
            500               505               510
Ile Arg Ile Asn His Ser Leu Gly Ser Leu Asp Ser Pro Pro Thr Cys
            515               520               525
Val Leu Pro Asp Ser Val Val Lys Pro Leu Pro Pro Ser Ser Val Lys
            530               535               540
Ala Glu Ile Thr Ile Asn Ile Gly Leu Leu Lys Ile Ser Trp Glu Lys
545               550               555                   560
Pro Val Phe Pro Glu Asn Asn Leu Gln Phe Gln Ile Arg Tyr Gly Leu
            565               570                   575
Ser Gly Lys Glu Val Gln Trp Lys Met Tyr Glu Val Tyr Asp Ala Lys
            580               585               590
Ser Lys Ser Val Ser Leu Pro Val Pro Asp Leu Cys Ala Val Tyr Ala
            595               600               605
Val Gln Val Arg Cys Lys Arg Leu Asp Gly Leu Gly Tyr Trp Ser Asn
            610               615               620
Trp Ser Asn Pro Ala Tyr Thr Val Val Met Asp Ile Lys Val Pro Met
625               630               635                   640
Arg Gly Pro Glu Phe Trp Arg Ile Ile Asn Gly Asp Thr Met Lys Lys
            645               650                   655
Glu Lys Asn Val Thr Leu Leu Trp Lys Pro Leu Met Lys Asn Asp Ser
            660               665                   670
Leu Cys Ser Val Gln Arg Tyr Val Ile Asn His His Thr Ser Cys Asn
            675               680               685
Gly Thr Trp Ser Glu Asp Val Gly Asn His Thr Lys Phe Thr Phe Leu
            690               695               700
Trp Thr Glu Gln Ala His Thr Val Thr Val Leu Ala Ile Asn Ser Ile
705               710               715                   720
Gly Ala Ser Val Ala Asn Phe Asn Leu Thr Phe Ser Trp Pro Met Ser
            725               730                   735
Lys Val Asn Ile Val Gln Ser Leu Ser Ala Tyr Pro Leu Asn Ser Ser
            740               745                   750
Cys Val Ile Val Ser Trp Ile Leu Ser Pro Ser Asp Tyr Lys Leu Met
            755               760                   765
Tyr Phe Ile Ile Glu Trp Lys Asn Leu Asn Glu Asp Gly Glu Ile Lys
            770               775               780
```

```
Trp Leu Arg Ile Ser Ser Ser Val Lys Lys Tyr Tyr Ile His Asp His
785                     790                 795                 800
Phe Ile Pro Ile Glu Lys Tyr Gln Phe Ser Leu Tyr Pro Ile Phe Met
                805                 810                 815
Glu Gly Val Gly Lys Pro Lys Ile Ile Asn Ser Phe Thr Gln Asp Asp
                820                 825                 830
Ile Glu Lys His Gln Ser Asp
                835


<210> 10
<211> 820
<212> PRT
<213> Homo sapiens

<220>
<221> SOURCE
<222> 1..820
<223> /mol_type="protein"
      /note="Leptinrezeptor Isoform B Fragment Thr20 - Asp839"
      /organism="Homo sapiens"

<400> 10
Thr Ala Phe Asn Leu Ser Tyr Pro Ile Thr Pro Trp Arg Phe Lys Leu
1               5                   10                  15
Ser Cys Met Pro Pro Asn Ser Thr Tyr Asp Tyr Phe Leu Leu Pro Ala
            20                  25                  30
Gly Leu Ser Lys Asn Thr Ser Asn Ser Asn Gly His Tyr Glu Thr Ala
            35                  40                  45
Val Glu Pro Lys Phe Asn Ser Ser Gly Thr His Phe Ser Asn Leu Ser
    50                  55                  60
Lys Thr Thr Phe His Cys Cys Phe Arg Ser Glu Gln Asp Arg Asn Cys
65                  70                  75                  80
Ser Leu Cys Ala Asp Asn Ile Glu Gly Lys Thr Phe Val Ser Thr Val
                85                  90                  95
Asn Ser Leu Val Phe Gln Gln Ile Asp Ala Asn Trp Asn Ile Gln Cys
            100                 105                 110
Trp Leu Lys Gly Asp Leu Lys Leu Phe Ile Cys Tyr Val Glu Ser Leu
        115                 120                 125
Phe Lys Asn Leu Phe Arg Asn Tyr Asn Tyr Lys Val His Leu Leu Tyr
    130                 135                 140
Val Leu Pro Glu Val Leu Glu Asp Ser Pro Leu Val Pro Gln Lys Gly
145                 150                 155                 160
Ser Phe Gln Met Val His Cys Asn Cys Ser Val His Glu Cys Cys Glu
            165                 170                 175
Cys Leu Val Pro Val Pro Thr Ala Lys Leu Asn Asp Thr Leu Leu Met
            180                 185                 190
Cys Leu Lys Ile Thr Ser Gly Gly Val Ile Phe Gln Ser Pro Leu Met
        195                 200                 205
Ser Val Gln Pro Ile Asn Met Val Lys Pro Asp Pro Pro Leu Gly Leu
    210                 215                 220
His Met Glu Ile Thr Asp Asp Gly Asn Leu Lys Ile Ser Trp Ser Ser
225                 230                 235                 240
Pro Pro Leu Val Pro Phe Pro Leu Gln Tyr Gln Val Lys Tyr Ser Glu
            245                 250                 255
Asn Ser Thr Thr Val Ile Arg Glu Ala Asp Lys Ile Val Ser Ala Thr
            260                 265                 270
Ser Leu Leu Val Asp Ser Ile Leu Pro Gly Ser Ser Tyr Glu Val Gln
        275                 280                 285
Val Arg Gly Lys Arg Leu Asp Gly Pro Gly Ile Trp Ser Asp Trp Ser
    290                 295                 300
Thr Pro Arg Val Phe Thr Thr Gln Asp Val Ile Tyr Phe Pro Pro Lys
305                 310                 315                 320
Ile Leu Thr Ser Val Gly Ser Asn Val Ser Phe His Cys Ile Tyr Lys
            325                 330                 335
Lys Glu Asn Lys Ile Val Pro Ser Lys Glu Ile Val Trp Trp Met Asn
            340                 345                 350
Leu Ala Glu Lys Ile Pro Gln Ser Gln Tyr Asp Val Val Ser Asp His
            355                 360                 365
```

```
Val Ser Lys Val Thr Phe Phe Asn Leu Asn Glu Thr Lys Pro Arg Gly
    370                     375                 380
Lys Phe Thr Tyr Asp Ala Val Tyr Cys Cys Asn Glu His Glu Cys His
    385                 390                 395                 400
His Arg Tyr Ala Glu Leu Tyr Val Ile Asp Val Asn Ile Asn Ile Ser
                405                     410                 415
Cys Glu Thr Asp Gly Tyr Leu Thr Lys Met Thr Cys Arg Trp Ser Thr
                420                     425             430
Ser Thr Ile Gln Ser Leu Ala Glu Ser Thr Leu Gln Leu Arg Tyr His
            435                     440                 445
Arg Ser Ser Leu Tyr Cys Ser Asp Ile Pro Ser Ile His Pro Ile Ser
    450                     455                 460
Glu Pro Lys Asp Cys Tyr Leu Gln Ser Asp Gly Phe Tyr Glu Cys Ile
465                     470                 475                 480
Phe Gln Pro Ile Phe Leu Leu Ser Gly Tyr Thr Met Trp Ile Arg Ile
                485                     490                 495
Asn His Ser Leu Gly Ser Leu Asp Ser Pro Pro Thr Cys Val Leu Pro
            500                     505             510
Asp Ser Val Val Lys Pro Leu Pro Pro Ser Ser Val Lys Ala Glu Ile
            515                     520             525
Thr Ile Asn Ile Gly Leu Leu Lys Ile Ser Trp Glu Lys Pro Val Phe
            530                     535             540
Pro Glu Asn Asn Leu Gln Phe Gln Ile Arg Tyr Gly Leu Ser Gly Lys
545                     550                 555                 560
Glu Val Gln Trp Lys Met Tyr Glu Val Tyr Asp Ala Lys Ser Lys Ser
                565                     570                 575
Val Ser Leu Pro Val Pro Asp Leu Cys Ala Val Tyr Ala Val Gln Val
                580                     585                 590
Arg Cys Lys Arg Leu Asp Gly Leu Gly Tyr Trp Ser Asn Trp Ser Asn
    595                     600                 605
Pro Ala Tyr Thr Val Val Met Asp Ile Lys Val Pro Met Arg Gly Pro
610                     615                 620
Glu Phe Trp Arg Ile Ile Asn Gly Asp Thr Met Lys Lys Glu Lys Asn
625                     630                 635                 640
Val Thr Leu Leu Trp Lys Pro Leu Met Lys Asn Asp Ser Leu Cys Ser
                645                     650                 655
Val Gln Arg Tyr Val Ile Asn His His Thr Ser Cys Asn Gly Thr Trp
                660                     665                 670
Ser Glu Asp Val Gly Asn His Thr Lys Phe Thr Phe Leu Trp Thr Glu
            675                     680                 685
Gln Ala His Thr Val Thr Val Leu Ala Ile Asn Ser Ile Gly Ala Ser
    690                     695                 700
Val Ala Asn Phe Asn Leu Thr Phe Ser Trp Pro Met Ser Lys Val Asn
705                     710                 715                 720
Ile Val Gln Ser Leu Ser Ala Tyr Pro Leu Asn Ser Ser Cys Val Ile
                725                     730                 735
Val Ser Trp Ile Leu Ser Pro Ser Asp Tyr Lys Leu Met Tyr Phe Ile
                740                     745                 750
Ile Glu Trp Lys Asn Leu Asn Glu Asp Gly Glu Ile Lys Trp Leu Arg
            755                     760                 765
Ile Ser Ser Ser Val Lys Lys Tyr Tyr Ile His Asp His Phe Ile Pro
    770                     775                 780
Ile Glu Lys Tyr Gln Phe Ser Leu Tyr Pro Ile Phe Met Glu Gly Val
785                     790                 795                 800
Gly Lys Pro Lys Ile Ile Asn Ser Phe Thr Gln Asp Asp Ile Glu Lys
                805                     810                 815
His Gln Ser Asp
                820
```